# EUROPÄISCHE PATENTSCHRIFT

(11) **EP 4 581 037 B1**
(45) Veröffentlichungstag und Bekanntmachung des Hinweises auf die Patenterteilung: **12.08.2026**
(21) Anmeldenummer: 22772451.5
(22) Anmeldetag: 29.08.2022
(51) Int. Cl.: C07F 1/08, C07D 209/94, C09K 11/06, H10K 50/11, H10K 85/00, H10K 85/30, H10K 50/80

(54) **EMITTERMATERIAL FÜR OLEDS**
EMITTER MATERIAL FOR OLEDS
MATÉRIAU ÉMETTEUR POUR OLED

(43) Veröffentlichungstag der Anmeldung: 09.07.2025
(73) Patentinhaber: Technische Universität Dortmund, 44227 Dortmund (DE)
(72) Erfinder: STEFFEN, Andreas, 33104 Paderborn (DE); MUTHIG, André, 44225 Dortmund (DE); FERSCHKE, Thomas, 97084 Würzburg (DE); PFLAUM, Jens, 97337 Dettelbach (DE); BJÖRN, Ewald, 97074 Würzburg (DE)
(74) Vertreter: Michalski Hüttermann & Partner mbB
(86) Internationale Anmeldenummer: PCT/EP2022/073938
(87) Internationale Veröffentlichungsnummer: WO 2024/046539

(56) Entgegenhaltungen:
- GERNERT MARKUS ET AL: "Cyclic (Amino)(aryl)carbenes Enter the Field of Chromophore Ligands: Expanded [pi] System Leads to Unusually Deep Red Emitting Cu I Compounds", JOURNAL OF THE AMERICAN CHEMICAL SOCIETY, vol. 142, no. 19, 17 April 2020 (2020-04-17), pages 8897 - 8909, XP93033249, DOI: 10.1021/jacs.0c02234
- GERNERT MARKUS ET AL: "Cyclic (Amino)(aryl)carbenes Enter the Field of Chromophore Ligands: Expanded [pi] System Leads to Unusually Deep Red Emitting Cu I Compounds", JOURNAL OF THE AMERICAN CHEMICAL SOCIETY, vol. 142, no. 19, 17 April 2020 (2020-04-17), pages 8897 - 8909, XP093033249, ISSN: 0002-7863, DOI: 10.1021/jacs.0c02234

## Beschreibung

Die vorliegende Erfindung betrifft eine Emitterschicht für eine OLED, welche beispielsweise zirkular polarisiertes Licht emittiert, wobei die Emitterschicht einen definierten Metallkomplex aufweist. Die vorliegende Erfindung betrifft ferner eine OLED aufweisend eine derartige Emitterschicht, ein Verfahren zum Erzeugen von Licht sowie die Verwendung eines Metallkomplexes zum Erzeugen von Licht in einer Emitterschicht einer OLED. Darüber hinaus umfasst die vorliegende Erfindung einen Liganden für einen entsprechenden Metallkomplex.

OLEDs sind heutzutage für eine Vielzahl von Anwendungen bekannt. Insbesondere bei TVs oder Displays, allgemein bei Anzeigegeräten, bieten sie aufgrund einer energiearmen Betriebsweise bei gleichzeitig guter Bildqualität mit hohem Kontrast Vorteile.

Displays auf Basis von OLEDs sind mit Filtern versehen, die die Reflexion von anderen Lichtquellen, wie etwa Tageslicht, verringern und somit einen Kontrastverlust vermeiden sollen. Als Filter werden lineare Polarisatoren kombiniert mit Viertelwellenplatten eingesetzt, wodurch allerdings nur noch ca. 50% des von der Emitterschicht emittierten Lichts passieren und vom Benutzer erfasst werden können. Daher wird für entsprechende Helligkeit (Brightness) eine hohe elektrische Leistung benötigt, um den Lichtverlust auszugleichen. Das von chiralen Emitter-Materialien generierte zirkular polarisierte Licht (circular polarized luminescence, CPL) mit hohem Dissymetrie-Faktor g_{Lum} hingegen kann die Filter zu 100% passieren, wodurch eine deutlich höhere Energieeffizienz erreicht werden kann. Diese sind jedoch im Stand der Technik noch nicht ausreichend bekannt.

Effiziente kommerzielle OLEDs benötigen derzeit phosphoreszierende Triplett-Emitter, zumeist in Form von Metallkomplexen der teuren Elemente Iridium oder Platin, um alle gebildeten Exzitonen der Elektron-Loch-Rekombination effizient in Licht umzuwandeln. Eine Alternative stellt die Lumineszenz über thermisch aktivierte verzögerte Fluoreszenz dar (TADF), die mit Komplexen des deutlich günstigeren Elementes Kupfer oder günstigen organischen Verbindungen realisiert werden kann. Wohingegen grün emittierende OLEDs gute Performance zeigen, ist eine effiziente Elektrolumineszenz im tief roten Farbbereich immer noch eine Herausforderung.

Der Einsatz von zyklischen (Amino)(Aryl) Carbenen als chromophore Liganden wird beispielsweise in Gernert Markus et al: "Cyclic (Amino) (aryl) carbenes Enter the Field of Chromophore Ligands: Expanded [pi] System Leads to Unusually Deep Red Emitting Cu I Compounds", JOURNAL OF THE AMERICAN CHEMICAL SOCIETY, Bd. 142, Nr. 19, 17. April 2020 (2020-04-17), Seiten 8897-8909, XP93033249, ISSN: 0002-7863, DOI: 10.1021/jacs.0c02234 beschrieben.

Es ist somit die Aufgabe der vorliegenden Erfindung wenigstes einen Nachteil des Stands der Technik zumindest teilweise zu überwinden. Insbesondere ist es die Aufgabe der vorliegenden Erfindung, eine Emitterschicht bereitzustellen, durch welche eine effiziente Elektrolumineszenz im tief roten Farbbereich möglich ist.

Diese Aufgabe wird zumindest zum Teil gelöst durch eine Verbindung gemäß Anspruch 1. Diese Aufgabe wird ferner zumindest zum Teil gelöst durch eine Verwendung gemäß Anspruch 8, durch einen Metallkomplex nach Anspruch 9, durch eine Verwendung nach Anspruch 12, durch eine Emitterschicht für eine organische Leuchtdiode mit den Merkmalen des Anspruchs 13, durch eine organische Leuchtdiode mit den Merkmalen des Anspruchs 14sowie durch ein Verfahren mit den Merkmalen des Anspruchs 15. Bevorzugte Ausgestaltungen der Erfindung sind in den Unteransprüchen, in der Beschreibung oder den Figuren beschrieben, wobei weitere in den Unteransprüchen oder in der Beschreibung oder den Figuren beschriebene oder gezeigte Merkmale einzeln oder in einer beliebigen Kombination einen Gegenstand der Erfindung darstellen können, wenn sich aus dem Kontext nicht eindeutig das Gegenteil ergibt.

Beschrieben wird eine Paracyclophan-Carben-Verbindung, dadurch gekennzeichnet, dass die Paracyclophan-Carben-Verbindung der folgenden Struktur (I) entspricht: 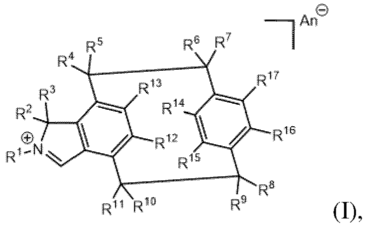 wobei
R¹ ausgewählt ist aus Aryl, Heteroaryl, Alkyl, Perfluoralkyl, Perfluoraryl, wobei
R² und R³ gleich oder unterschiedlich sind und unabhängig voneinander ausgewählt sind aus Aryl, Heteroaryl, Alkyl, Alkenyl, Alkinyl, Perfluoralkyl, Perfluoraryl, wobei
R⁴, R⁵, R⁶, R⁷, R⁸, R⁹, R¹⁰, R¹¹ gleich oder unterschiedlich sind und unabhängig voneinander ausgewählt sind aus Wasserstoff, Deuterium, Halogen, Silyl, Alkyl, Alkenyl, Alkinyl, Perfluoralkyl, Aryl, Heteroaryl, Alkoxy, wobei
R¹² R¹³, R¹⁴, R¹⁵, R¹⁶, R¹⁷ gleich oder unterschiedlich sind und unabhängig voneinander ausgewählt sind aus Wasserstoff, Deuterium, Halogen, Alkyl, Alkenyl, Alkinyl, Perfluoralkyl, Aryl, Heteroaryl, Alkoxy, COR¹⁸, CO₂R¹⁹, CONR²⁰, Cyano, Nitro, SO₃R²¹, PO₃R²²₂, wobei R¹⁸ R¹⁹, R²⁰, R²¹, R²² gleich oder unterschiedlich sind und unabhängig voneinander ausgewählt sind aus Wasserstoff, Deuterium, Halogen, Silyl, Alkyl, Alkenyl, Alkinyl, Perfluoralkyl, Aryl, Heteroaryl, Alkoxy, und wobei
An⁻ ein Anion ist.

Die in der Struktur (I) gezeigte Verbindung ist als Salz gezeigt, wobei das Kation als Ligand dient, der eine Bindung zu einem Metall ausbilden kann. Insoweit ist es für den Fachmann unmittelbar verständlich, dass, wenn im Rahmen der vorliegenden Erfindung von einem entsprechenden Liganden gesprochen wird, das Paracyclophan-Carben-Kation gemeint ist, welches dann wie für Liganden üblich an ein Metallatom gebunden werden kann oder gebunden ist.

Grundsätzlich soll beispielsweise ein C1 Alkyl einen Alkylrest mit einem Kohlenstoffatom bezeichnen, wobei die entsprechenden Bezeichnungen für die weiteren Reste entsprechend zu verstehen sind, so dass etwa ein C12 Alkoxy eine Alkoxygruppe mit 12 Kohelnstoffatomen bezeichnet.

Ferner beschreibt der Begriff An⁻ grundsätzlich ein Anion unabhängig von seiner Wertigkeit. Dabei sollen das Paracyclophan-Carben-Kation mit dem Anion ein neutrales Salz zu bilden. In Abhängigkeit des gwählten Anions können somit beispielsweise ein, zwei oder mehr Kationen vorliegen, um die Paracyclophan-Carben-Verbindung gemäß Struktur (I) als in Summe neutrales Salz zu bilden.

Eine derartige Paracyclophan-Carben-Verbindung gemäß Struktur (I) dient in besonders vorteilhafter Weise dazu, einen Liganden für einen Metallkomplex auszubilden, der als Emittermaterial in einer OLED dient.

Hinsichtlich einer Verwendung in einer OLED stellt die Lumineszenz über thermisch aktivierte verzögerte Fluoreszenz (TADF) eine Alternative zu Lösungen aus dem Stand der Technik dar, die mit Komplexen sehr günstiger Metalle, wie etwa Kupfer oder auch mit günstigen organischen Verbindungen realisiert werden kann. Wohingegen grün emittierende OLEDs gute Performance zeigen, ist eine effiziente Elektrolumineszenz im tief roten Farbbereich immer noch eine Herausforderung.

Durch den erfindungsgemäßen Liganden werden aus kommerziellen Ausgangsstoffen im Gegensatz zu etablierten Iridium-Emittern sehr günstig herzustellende racemische Metallkomplexe, wie etwa Kupfer(I)-Komplexe, beschrieben, die aufgrund von TADF die für molekulare Emitter bisher höchsten berichteten Strahlungskonstanten im roten Bereich von bis zu 2x10⁶ s⁻¹ aufweisen und auch erfolgreich in einer Test-OLED angewendet wurden.

Bei einer Verwendung als Liganden von Metallkomplexen in Emittermaterielien für OLEDs können die beschriebenen Liganden somit eine sehr hohe Lichtausbeute ermöglichen und damit die Qualität von OLEDs deutlich erhöhen. Darüber hinaus lassen sie sich mit einem vergleichsweise sehr geringen Energieeinsatz betreiben.

Aufgrund ihrer chiralen Natur eignen sich diese Verbindungen in enantiomerenreiner Form prinzipiell auch zur Verwendung in OLEDs basierend auf zirkular polarisierter Lumineszenz (CPL), um die Antireflektionsfilter der Displays mit maximaler Effizienz zu passieren. Diese neue Emitter-Klasse zeigt damit das Potential für kommerzielle energieeffiziente Anwendungen von Interesse zu sein. Die positiven Eigenschaften lassen sich gegebenenfalls bei weiterer Optimierung der molekularen Struktur als auch des OLED-Aufbaus vermutlich noch weiter verbessern.

Grundsätzlich bietet eine Emitterschicht gemäß der vorliegenden Erfindung somit die Möglichkeit, für jeweilige Anwendungen maßgeschneidert zu werden.

Darüber hinaus können durch die möglichen Synthesewege die Herstellbarkeit deutlich günstiger und/oder einfacher gegenüber den im Stand der Technik beispielsweise verwendeten Iridium- oder Plateinkomplexen sein.

Somit lässt sich erfindungsgemäß ein energiearmer Betrieb einer OLED kombinieren mit einer hohen Lichtausbeute und somit einer hohen Qualität bei gleichzeitig moderaten Kosten, wobei insbesondere eine hohe Lichtausbeute im roten Spektralbereich möglich ist. Hinsichtlich der Paracyclophan-Carben-Verbindung, welche als Ligand in einem Metallkomplex einer Emitterschicht einer OLED dienen kann, kann es besonders bevorzugt sein, dass
R¹ ausgewählt ist aus C5 bis C18 Aryl, C5 bis C18 Heteroaryl, C1 bis C18 Alkyl, C1 bis C18 Perfluoralkyl, C5 bis C18 Perfluoraryl, wobei
R² und R³ gleich oder unterschiedlich sind und unabhängig voneinander ausgewählt sind aus C5 bis C18 Aryl, C5 bis C18 Perfluoraryl, C5 bis C18 Heteroaryl, C1 bis C12 Alkyl, C1 bis C12 Alkenyl, C1 bis C12 Alkinyl, C1 bis C12 Perfluoralkyl, wobei
R⁴, R⁵, R⁶, R⁷, R⁸, R⁹, R¹⁰, R¹¹ gleich oder unterschiedlich sind und unabhängig voneinander ausgewählt sind aus Wasserstoff, Deuterium, Halogen, Silyl, C1 bis C12 Alkyl, C1 bis C12 Alkenyl, C1 bis C12 Alkinyl und C1 bis C12 Perfluoralkyl, C5 bis C18 Aryl, C5 bis C18 Perfluoraryl, C5 bis C18 Heteroaryl, C1 bis C12 Alkoxy, wobei
R¹² R¹³, R¹⁴, R¹⁵, R¹⁶, R¹⁷ gleich oder unterschiedlich sind und unabhängig voneinander ausgewählt sind aus Wasserstoff, C1 bis C18 Alkyl, C1 bis C18 Alkenyl, C1 bis C18 Alkinyl, und C1 bis C18 Perfluoralkyl, C5 bis C18 Aryl, C5 bis C18 Perfluoraryl, C5 bis C18 Heteroaryl, C1 bis C18 Alkoxy, COR¹⁸, CO₂R¹⁹, CONR²⁰, Cyano, Nitro, SO₃R²¹, PO₃R²²₂, wobei
R¹⁸ R¹⁹, R²⁰, R²¹, R²² gleich oder unterschiedlich sind und unabhängig voneinander ausgewählt sind aus Wasserstoff, Deuterium, Halogen, Silyl, C1 bis C18 Alkyl, C1 bis C18 Alkenyl, C1 bis C18 Alkinyl, C1 bis C18 Perfluoralkyl, C5 bis C18 Aryl, C5 bis C18 Heteroaryl, C1 bis C18 Alkoxy, und wobei
An⁻ ein Anion ist, das ausgewählt ist aus Halogenid, Triflat, Sulfat, Phosphat, Silikat, Tetrafluoroborat, Hexafluorophosphat, Tetraarylborat.

Besonders bevorzugt kann R¹ ausgewählt sein aus C9 bis C18 Aryl, C9 bis C18 Heteroaryl, C1 bis C12 Alkyl, C1 bis C12 Perfluoralkyl, C9 bis C18 Perfluoraryl, wobei R² und R³ gleich oder unterschiedlich sind und unabhängig voneinander ausgewählt sind aus C5 bis C6 Aryl und C5 bis C6 Heteroaryl,
R⁴, R⁵, R⁶, R⁷, R⁸, R⁹, R¹⁰, R¹¹ gleich oder unterschiedlich sind und unabhängig voneinander ausgewählt sind aus Wasserstoff, Deuterium, Halogen und Silyl, C1 bis C4 Alkyl, C1 bis C4 Alkenyl, C1 bis C4 Alkinyl und C1 bis C4 Perfluoralkyl, C5 bis C6 Aryl, C5 bis C6 Heteroaryl, C5 bis C6 Perfluoraryl, C1 bis C4 Alkoxy, wobei
R¹² R¹³, R¹⁴, R¹⁵, R¹⁶, R¹⁷ gleich oder unterschiedlich sind und unabhängig voneinander ausgewählt sind aus Wasserstoff, C1 bis C4 Alkyl, C1 bis C4 Alkenyl, C1 bis C4 Alkinyl, und C1 bis C4 Perfluoralkyl, C5 bis C6 Aryl, C5 bis C6 Perfluoraryl, C5 bis C6 Heteroaryl, C1 bis C4 Alkoxy, COR¹⁸, CO₂R¹⁹, CONR²⁰, Cyano, Nitro, SO₃R²¹, PO₃R²²₂, wobei
R¹⁸ R¹⁹, R²⁰, R²¹, R²² gleich oder unterschiedlich sind und unabhängig voneinander ausgewählt sind aus Wasserstoff, Deuterium, Halogen, Silyl, C1 bis C4 Alkyl, C1 bis C4 Alkenyl, C1 bis C4 Alkinyl, C1 bis C4 Perfluoralkyl, C5 bis C12 Aryl, C5 bis C12 Heteroaryl, C1 bis C4 Alkoxy, und wobei
An⁻ ein Anion ist, das ausgewählt ist aus Halogenid, Triflat, Sulfat, Phosphat, Silikat, Tetrafluoroborat, Hexafluorophosphat, Tetraarylborat.

Besonders bevorzugt kann R¹ ein Dialkylphenyl sein. Beispielsweise kann das Dialkylphenyl 2,6-Di-iso-propylphenyl sein.

Es kann weiterhin bevorzugt sein, dass R² und R³ Phenyl sind. Grundsätzlich können die Phenlyringe substituiert sein, es ist jedoch auch möglich, dass die Phenlyringe unsubstituiert sind.

Weiter bevorzugt kann es vorgesehen sein, dass R⁴, R⁵, R⁶, R⁷, R⁸, R⁹, R¹⁰ und R¹¹ Wasserstoff sind.

Zusätzlich oder alternativ kann es von Vorteil sein, dass R¹² R¹³, R¹⁴, R¹⁵, R¹⁶ und R¹⁷ Wasserstoff, sind.

In einer spezifischen Ausgestaltung der vorliegenden Erfindung kann es vorgesehen sein, dass Paracyclophan-Carben-Verbindung die folgende Struktur (II) aufweist:

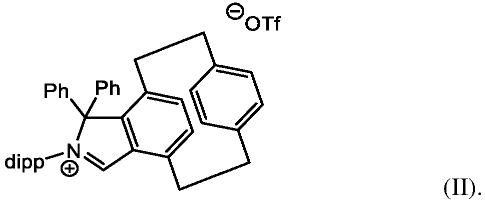

Bei dieser Struktur soll der Rest "dipp" 2,6-Di-iso-propylphenyl meinen und soll das Anion OTF⁻ eine Triflatgruppe sein, die auch als Trifluoromethansulfonat-Gruppe bezeichnet werden kann.

Dem Vorstehenden folgend ist ein Gegenstand der vorliegenden Erfindung ferner eine Verwendung einer Paracyclophan-Carben-Verbindung, wie diese vorstehend beschrieben ist, zur Herstellung eines Metallkomplexes für ein Emittermaterial einer Emitterschicht einer organischen Leuchtdiode.

Zusammenfassend lässt sich dadurch wie vorstehend im Hinblick auf die Paracyclophan-Carben-Verbindung im Detail beschrieben ein energiearmer Betrieb einer OLED kombinieren mit einer hohen Lichtausbeute und somit einer hohen Qualität bei gleichzeitig moderaten Kosten, wobei insbesondere eine hohe Lichtausbeute im roten Spektralbereich möglich ist.

Hinsichtlich weiterer Vorteile und technischer Merkmale der Paracyclophan-Carben-Verbindung wie auch ihrer Verwendung wird auf die Beschreibung des Metallkomplexes, seiner Verwendung als Emittermaterial in einer Emitterschicht, der Emitterschicht, der organischen Leuchtdiode, des Verfahrens, der Figuren wie auch der Beschreibung der Figuren verwiesen.

Beschrieben wird ferner ein Metallkomplex. Der Metallkomplex ist dadurch gekennzeichnet, dass dieser der folgenden Struktur (III) entspricht:

L¹Me[L²]ₘ[L³]ₘ (III),

wobei L¹ ein Paracyclophan-Carben-Ligand wie vorstehend beschrieben ist, wobei L² und L³ gleich oder unterschiedlich sind und unabhängig voneinander ausgewählt sind aus NR²³₂, NR²⁴₃, PR²⁵₂, PR²⁶₃, OR²⁷₂, OR²⁸, SR²⁹, SR³⁰₂, SR³¹, SeR³²₂, SeR³⁴, TeR³⁵₂, TeR³⁶, Silyl, Aryl, Heteroaryl, Alkenyl, Alkinyl, Isonitril, Cyclopentadienyl, Halogen, wobei
m eine Zahl ist ausgewählt aus 0, 1 oder 2 mit der Maßgabe, dass wenigstens ein m ungleich null ist, wobei
R²³, R²⁴, R²⁵, R²⁶, R²⁷, R²⁸, R²⁹, R³⁰, R³¹, R³², R³³, R³⁴, R³⁵, R³⁶ gleich oder unterschiedlich sind und unabhängig voneinander ausgewählt sind aus Aryl, Heteroaryl, Alkenyl, Alkinyl, Alkyl, Perfluoralkyl, Perfluoraryl; und wobei
Me ein Metall ist, das ausgewählt ist aus Kupfer, Silber und Gold.

Hinsichtlich der Beschreibung des Liganden L¹ als Paracyclophan-Carben-Ligand wird auf folgendes hingewiesen. Das Paracyclophan-Carben-basierte Salz wird, wie für Carbene in der Literatur umfassend beschrieben und allgemein bekannt, mit einer Base umgesetzt und der resultierende neutrale Carben-Ligand an das Metall gebunden. In anderen Worten kann die Paracyclophan-Carben-Verbindung auch als entsprechendes Ligandsalz mit Paracyclophan-Carben-Struktureinheit als Ligand bezeichnet werden.

Grundsätzlich kann an dem Metall des Metallkomplexes zusätzlich zu dem Ligand L¹ nur ein Ligand L2 aber kein Ligand L3, nur ein Ligand L3 aber kein Ligand L2 oder können beide Liganden L2 und L3 vorhanden sein. Darüber hinaus können die Liganden L2 und L3 von einander getrennt sein oder es kann ein Ligand L2 vorhanden sein, welcher mit zwei Bindungen an das Metall bindet und somit als überbrückender Ligand oder Chelatligand bezeichnet werden kann.

Ein derartiger Metallkomplex dient insbesondere als Emittermaterial in einer Emitterschicht einer organischen Leuchtdiode. Dadurch ergeben sich insbesondere die Vorteile, wonach eine effiziente Elektrolumineszenz insbesondere im tief roten Farbbereich ermöglicht wird. Dies ist bei Lösungen aus dem Stand der Technik oftmals noch eine Herausforderung.

Durch den erfindungsgemäßen Metallkomplex werden aus kommerziellen Ausgangsstoffen im Gegensatz zu etablierten Iridium-Emittern sehr günstig herzustellende racemische MetallKomplexe beschrieben, die aufgrund von TADF die für molekulare Emitter bisher höchsten berichteten Strahlungskonstanten im roten Bereich von bis zu 2x10⁶ s⁻¹ aufweisen und auch erfolgreich in einer Test-OLED angewendet wurden.

Zusammenfassend kann eine sehr hohe Lichtausbeute ermöglicht werden und damit die Qualität von OLEDs deutlich erhöht werden. Darüber hinaus lassen sie sich mit einem vergleichsweise sehr geringen Energieeinsatz betreiben.

Somit lässt sich erfindungsgemäß ein energiearmer Betrieb einer OLED kombinieren mit einer hohen Lichtausbeute und somit einer hohen Qualität bei gleichzeitig moderaten Kosten, wobei insbesondere eine hohe Lichtausbeute im roten Spektralbereich möglich ist.

Es kann bevorzugt sein, dass
L¹ ein Paracyclophan-Carben-Ligand wie vorstehend beschrieben ist, wobei
Me ein Metall ist, das ausgewählt ist aus Kupfer, Silber und Gold, und wobei
L² ein Ligand ist, der ausgewählt ist aus einem Heteroaryl und einem Halogen.

Beispielsweise kann es bevorzugt sein,
L¹ ein Paracyclophan-Carben-Ligand wie vorstehend beschrieben ist, wobei
Me Kupfer ist, und wobei
L² ein Ligand ist, der ausgewählt ist aus einem Carbazol-Liganden und einem Halogen.

Insbesondere unter Verwendung eines Kupferkomplexes lassen sich beispielsweise gegenüber Iridiumkomplexen aus dem Stand der Technik kostengünstige Komplexe ausbilden. Entsprechend können die die diesbezüglichen Vorteile besonders ausgeprägt sein.

In einer speziellen Ausgestaltung kann es vorgesehen sein, dass der Metallkomplex ausgewählt ist aus den Strukturen (IV), (V), (VI), (VII), (VIII) und (IX) wie nachfolgend gezeigt:

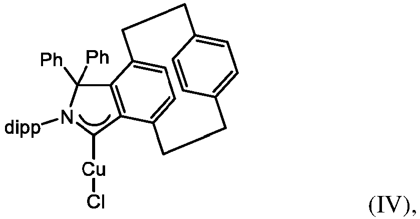

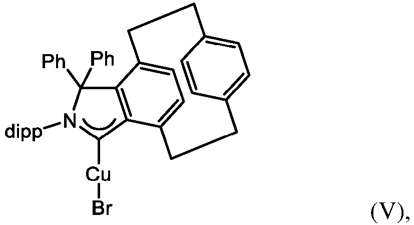

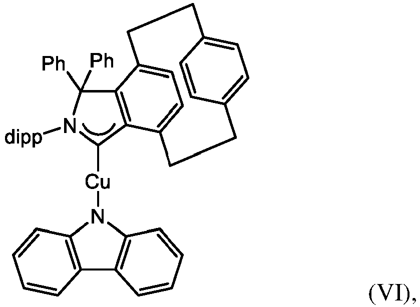

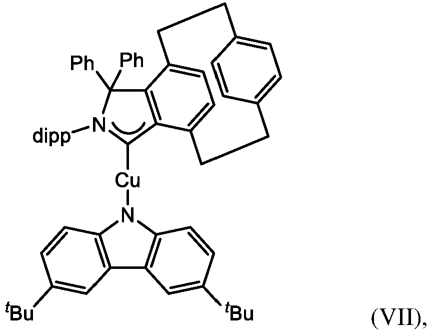

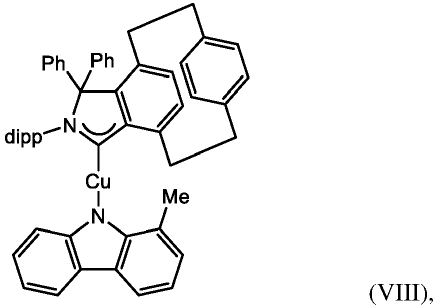

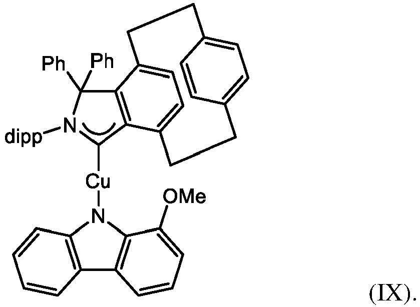

Dem Vorstehenden folgend ist ferner ein Gegenstand der vorliegenden Erfindung eine Verwendung eines Metallkomplexes wie vorstehend beschrieben als Emittermaterial in einer Emitterschicht einer organischen Leuchtdiode.

Zusammenfassend lässt sich dadurch wie vorstehend im Hinblick auf die Paracyclophan-Carben-Verbindung als auch den entsprechenden Metallkomplex im Detail beschrieben ein energiearmer Betrieb einer OLED kombinieren mit einer hohen Lichtausbeute und somit einer hohen Qualität bei gleichzeitig moderaten Kosten, wobei insbesondere eine hohe Lichtausbeute im roten Spektralbereich möglich ist.

Hinsichtlich weiterer Vorteile und technischer Merkmale des Metallkomplexes und seiner Verwendung als Emittermaterial in einer Emitterschicht wird auf die Beschreibung der Paracyclophan-Carben-Verbindung wie auch ihrer Verwendung, der Emitterschicht, der organischen Leuchtdiode, des Verfahrens, der Figuren wie auch der Beschreibung der Figuren verwiesen.

Beschrieben wird ferner eine Emitterschicht für eine organische Leuchtdiode, aufweisend einen Metallkomplex zum Emittieren von Licht, dadurch gekennzeichnet, dass der Metallkomplex ausgebildet ist, wie vorstehend beschrieben.

Beschrieben wird somit eine Emitterschicht für eine organische Leuchtdiode (OLED). Grundsätzlich kann die OLED aufgebaut sein, wie dies aus dem Stand der Technik bekannt ist. Es brauchen somit keine besonderen Anforderungen erfüllt werden, um die vorstehend beschriebene Emitterschicht in einer OLED umzusetzen.

Die Emitterschicht zeichnet sich dadurch aus, dass in dieser als aktives Emittermaterial ein Metallkomplex enthält, wie dieser vorstehend beschrieben ist. In einer allgemeinen Form enthält die Emitterschicht einen Metallkomplex, der der folgenden Struktur (III) entspricht:

L¹Me[L²]ₘ[L³]ₘ (III),
wobei L¹ ein Paracyclophan-Carben-Ligand ist, wie vorstehend beschrieben, wobei
L² und L³ gleich oder unterschiedlich sind und unabhängig voneinander ausgewählt sind aus NR²³₂, NR²⁴₃, PR²⁵₂, PR²⁶₃, OR²⁷₂, OR²⁸, SR²⁹, SR³⁰₂, SR³¹, SeR³²₂, SeR³⁴, TeR³⁵₂, TeR³⁶, Silyl, Aryl, Heteroaryl, Alkenyl, Alkinyl, Isonitril, Cyclopentadienyl, Halogen, wobei m eine Zahl ist ausgewählt aus 0, 1 oder 2 mit der Maßgabe, dass wenigstens ein m ungleich null ist, wobei
R²³, R²⁴, R²⁵, R²⁶, R²⁷, R²⁸, R²⁹, R³⁰, R³¹, R³², R³³, R³⁴, R³⁵, R³⁶ gleich oder unterschiedlich sind und unabhängig voneinander ausgewählt sind aus Aryl, Heteroaryl, Alkenyl, Alkinyl, Alkyl, Perfluoralkyl, Perfluoraryl; und wobei
Me ein Metall ist, das ausgewählt ist aus Kupfer, Silber und Gold.

Grundsätzlich kann es bevorzugt sein, dass
L¹ ein Paracyclophan-Carben-Ligand wie vorstehend beschrieben ist, wobei Me ein Metall ist, das ausgewählt ist aus Kupfer, Silber und Gold, und wobei L² ein Ligand ist, der ausgewählt ist aus einem Heteroaryl und einem Halogen.

Beispielsweise kann es bevorzugt sein,
L¹ ein Paracyclophan-Carben-Ligand wie vorstehend beschrieben ist, wobei
Me Kupfer ist, und wobei
L² ein Ligand ist, der ausgewählt ist aus einem Carbazol-Liganden und einem Halogen.

Insbesondere unter Verwendung eines Kupferkomplexes lassen sich beispielsweise gegenüber Iridiumkomplexen aus dem Stand der Technik kostengünstige Komplexe ausbilden. Entsprechend können die die diesbezüglichen Vorteile besonders ausgeprägt sein.

In einer speziellen Ausgestaltung kann es vorgesehen sein, dass der Metallkomplex ausgewählt ist aus den Strukturen (IV), (V), (VI), (VII), (VIII) und (IX) wie nachfolgend gezeigt:

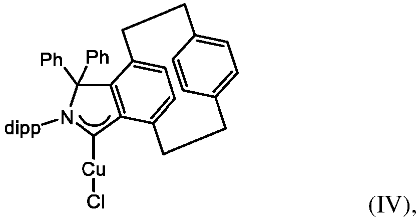

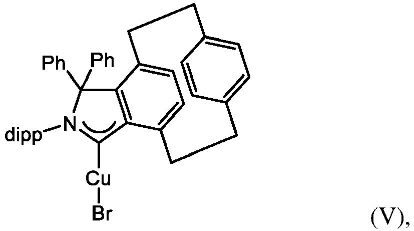

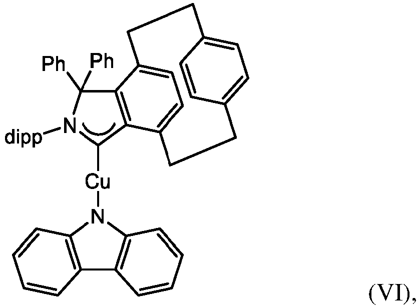

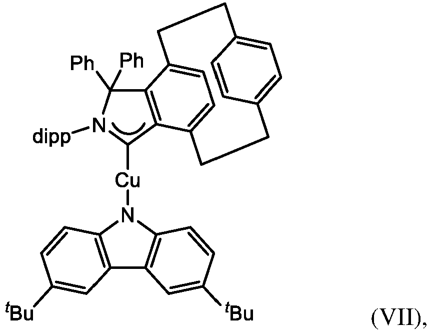

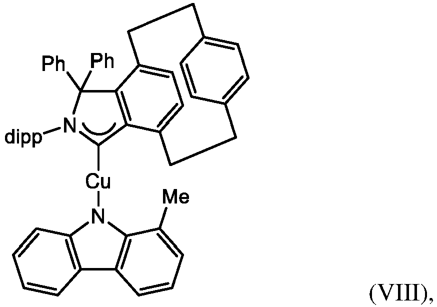

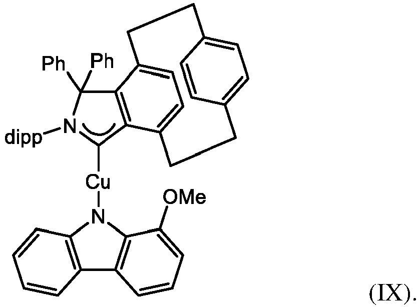

Zusammenfassend werden durch den erfindungsgemäßen Liganden aus kommerziellen Ausgangsstoffen im Gegensatz zu etablierten Iridium-Emittern sehr günstig herzustellende racemische Metallkomplexe, wie etwa Kupfer(I)-Komplexe, beschrieben, die aufgrund von TADF die für molekulare Emitter bisher höchsten berichteten Strahlungskonstanten im roten Bereich von bis zu 2x10⁶ s⁻¹ aufweisen und auch erfolgreich in einer Test-OLED angewendet wurden.

Bei einer Verwendung als Liganden von Metallkomplexen in Emittermaterielien für OLEDS können die beschriebenen Liganden somit eine sehr hohe Lichtausbeute ermöglichen und damit die Qualität von OLEDs deutlich erhöhen. Darüber hinaus lassen sie sich mit einem vergleichsweise sehr geringen Energieeinsatz betreiben.

Somit lässt sich erfindungsgemäß ein energiearmer Betrieb einer OLED kombinieren mit einer hohen Lichtausbeute und somit einer hohen Qualität bei gleichzeitig moderaten Kosten, wobei insbesondere eine hohe Lichtausbeute im roten Spektralbereich möglich ist.

Hinsichtlich weiterer Vorteile und technischer Merkmale der Emitterschicht wird auf die Beschreibung der Paracyclophan-Carben-Verbindung wie auch ihrer Verwendung, des Metallkomplexes, seiner Verwendung als Emittermaterial in einer Emitterschicht, der organischen Leuchtdiode, des Verfahrens, der Figuren wie auch der Beschreibung der Figuren verwiesen.

Weiterhin beschrieben ist eine organische Leuchtdiode (OLED), aufweisend eine Kathode, eine Emitterschicht, eine Lochleitungsschicht und eine Anode, dadurch gekennzeichnet, dass die Emitterschicht ausgebildet ist wie vorstehend beschrieben.

Entsprechend kann der das Emittermaterial bildende und in der Emitterschicht befindliche Metallkomplex der folgenden Struktur (III) entsprechen:

L¹Me[L²]ₘ[L³]ₘ (III),

wobei L¹ ein Paracyclophan-Carben-Ligand wie vorstehend beschrieben ist, wobei L² und L³ gleich oder unterschiedlich sind und unabhängig voneinander ausgewählt sind aus NR²³₂, NR²⁴₃, PR²⁵₂, PR²⁶₃, OR²⁷₂, OR²⁸, SR²⁹, SR³⁰₂, SR³¹, SeR³²₂, SeR³⁴, TeR³⁵₂, TeR³⁶, Silyl, Aryl, Heteroaryl, Alkenyl, Alkinyl, Isonitril, Cyclopentadienyl, Halogen, wobei
m eine Zahl ist ausgewählt aus 0, 1 oder 2 mit der Maßgabe, dass wenigstens ein m ungleich null ist, wobei
R²³, R²⁴, R²⁵, R²⁶, R²⁷, R²⁸, R²⁹, R³⁰, R³¹, R³², R³³, R³⁴, R³⁵, R³⁶ gleich oder unterschiedlich sind und unabhängig voneinander ausgewählt sind aus Aryl, Heteroaryl, Alkenyl, Alkinyl, Alkyl, Perfluoralkyl, Perfluoraryl; und wobei
Me ein Metall ist, das ausgewählt ist aus Kupfer, Silber und Gold.

Der Metallkomplex der Emitterschicht kann dabei weitergebildet sein, wie dies vorstehend mit Bezug auf die Beschreibung des Metallkomplexes im Detail beschrieben ist.

Wie vorstehend in größerem Detail in Hinblick auf die Emitterschicht beschrieben bieten sich durch eine Verwendung des Kupferkomplexes als Emittermaterial in der Emitterschicht die Vorteile eines energiearmen Betriebs einer entsprechend ausgestatteten OLED in Kombination mit einer hohen Lichtausbeute insbesondere im roten Spektralbereich, wobei zusätzlich eine kostengünstige Herstellbarkeit möglich ist.

Der Aufbau der OLED umfassend die Schichten Kathode, Emitterschicht, Lochleitungsschicht und Anode kann dabei grundsätzlich ausgebildet sein, wie dies aus dem Stand der Technik bekannt ist.

Beispielsweise kann die Kathode ein Metall beziehungsweise eine Metalllegierung aufweisen oder daraus bestehen, so dass die Kathode beziehungsweise deren Material eine geringe Elektronenaustrittsarbeit aufweist. Beispiele umfassen etwa Calcium, Aluminium, Barium, Ruthenium oder eine Magnesium-Silber-Legierung.

Die Anode kann beispielsweise ein metallisches Oxid sein, wie etwa Indium-Zinn-Oxid (ITO).

Die Lochleitungsschicht (hole transport layer, HTL) kann ein molekulares oder polymeres Material sein, wie beispielsweise Poly(3,4-ethylenedioxythiophen) Polystyrol-Sulfonat (PEDOT:PSS).

Die Emitterschicht weist als Emittermaterial insbesondere den vorstehend beschriebenen Kupferkomplex auf. Dieser liegt bevorzugt verdünnt, mit Konzentrationen von beispielsweise 1 - 10 Gew.-%, bevorzugt 1-5 Gew.-%, in einer geeigneten Matrix vor, wobei sich die vorstehend beschriebenen Anteile auf die gesamte Emitterschicht beziehen. Ein geeignetes Matrixmaterial kann beispielsweise 1,3-Bis(N-carbazolyl)benzol (mCP) sein.

Hinsichtlich des Matrixmaterials ist dieses grundsätzlich in einem weiten Rahmen wählbar, solange die Energieniveaus einen Energietransfer oder eine direkte Rekombination auf dem Emitter ermöglichen. Das genaue Material ist insbesondere zu wählen in Abhängigkeit von den gewählten Cu-Komplex-Energien, also etwa von den spezifisch gewählten Liganden.

Darüber hinaus kann der vorbeschriebene Schichtaufbau etwa auf einem Substrat, wie beispielsweise einem Glassubstrat, aufgebracht sein.

Die Herstellung und der Aufbau der OLED können grundsätzlich denen aus dem Stand der Technik entsprechen.

Grundsätzlich kann die OLED nur aus vorbeschriebenen Schichten bestehen, wobei jedoch auch weitere Schichten nicht ausgeschlossen sind, sondern von der vorliegenden Erfindung umfasst sein sollen. Beispielsweise können optimierte, dotierte Ladungstransportschichten oder Ladungs/Exzitonen-blockierenden Schichten denkbar sein. Die zusätzlichen Schichten können beispielsweise einer Energie-Maßschneiderung der aufeinanderfolgenden funktionellen Schichten, wie etwa Ausbalancieren der Ladungsträgerinjektionsassymmetrie, Erzeugen von gezielten Energiekaskaden um den Energieübertrag auf den Emitter zu optimieren, etc. dienen.

Es kann bevorzugt sein, dass die organische Leuchtdiode weiterhin einen Filter zum Reduzieren von Reflektionen externer Lichtquellen aufweist, wobei der Filter zumindest zum Teil undurchlässig ist für linear polarisiertes Licht.

Zusätzlich zu den vorbeschriebenen Schichten weist der Schichtaufbau beziehungsweise die OLED somit bevorzugt einen Filter beziehungsweise eine Filterkombination auf, die dazu dient, Reflektionen externer Lichtquellen zu reduzieren. Die Filterkombination besteht dabei insbesondere aus einem Linearpolarisator und einem Lambda/4-Plättchen. Eine derartige Filterkombination zeichnet sich somit dadurch aus, dass sie linear polarisiertes Licht zumindest zum Teil aus einem Strahlengang herausfiltert und dadurch etwa Tageslicht aus dem Strahlengang herausfiltert. Während bei der Emission standardmässger OLEDs deshalb auch ein Teil des erzeugten linear-polarisierten Lichts durch diese Filteranordnung blockiert wird und nicht zur Lichterzeugung beiträgt, kann mit den vorgeschlagenen Emittern, die insbesondere zirkular-polarisiertes Licht emittieren, prinzipiell jedes der erzeugten Lichtteilchen ausgekoppelt werden und zur Lichterzeugung beitragen. Dies führt dazu, dass Reflektionen von Tageslicht signifikant reduziert werden, während das intern erzeugte Licht vollständig die Filteranordnung passieren und damit den Kontrast eines von einer OLED ausgestrahlten Bildes insbesondere unter traditionell schwierigen Nutzungssituationen, wie etwa starke Sonneneinstrahlung im Außenbereich, deutlich verbessern kann.

Ein derartiger Filter kann dabei insbesondere bei einer OLED wie vorstehend beschrieben Vorteile mit sich bringen, da eine mit einem Linearpolarisator als Filter versehene OLED die Lichtausbeute von zirkular polarisiertem Licht nicht vermindert. Daher kann insbesondere bei einer erfindungsgemäßen OLED ein hochqualitatives Bild ermöglicht werden.

Hinsichtlich weiterer Vorteile und technischer Merkmale der organischen Leuchtdiode wird auf die Beschreibung der Paracyclophan-Carben-Verbindung wie auch ihrer Verwendung, des Metallkomplexes, seiner Verwendung als Emittermaterial in einer Emitterschicht, der Emitterschicht, des Verfahrens, der Figuren wie auch der Beschreibung der Figuren verwiesen.

Beschrieben wird ferner ein Verfahren zum Erzeugen von insbesondere zirkular polarisiertem Licht durch eine organische Leuchtdiode, aufweisend die Verfahrensschritte:
a) Bereitstellen einer organischen Leuchtdiode, wie diese vorstehend beschrieben ist; und
b) Anlegen einer Spannung zwischen der Anode und der Kathode zur Injektion von Ladungsträgern und zur Erzeugung von insbesondere zirkular polarisiertem Licht.

Mit Bezug auf den Verfahrensschritt a) und dabei insbesondere hinsichtlich der Aus- und Weiterbildung der OLED beziehungsweise insbesondere des Emittermaterials in der Emitterschicht wird vollumfämglich auf die entsprechende Beschreibung der OLED beziehungsweise der Emitterschicht Bezug genommen.

Durch das Anlegen einer Spannung zwischen Anode und Kathode, was in dem für einen Fachmann für OLEDs bekannten Rahmen realisierbar ist, kann das Emittermaterial in der Emitterschicht dazu angeregt werden, insbesondere zirkular polarisiertes Licht zu emittieren. Dies ist in überraschender Weise mit dem vorstehend beschriebenen Kupferkomplex mit hoher Lichtausbeute möglich, was signifikante Vorteile mit sich bringt.

Die OLED kann dabei in einem für etablierte OLED Architekturen üblichen Spannungsbereich von wenigen Volt und Stromdichtebereich von bis zu 1 A/cm² betrieben werden, beispielsweise bei 10 mA/cm². Dadurch kann die OLED mit der insbesondere zirkular emittierenden aktiven Schicht durch etablierte elektrische Schaltkreise angesteuert und in bestehende Aufbauten (z.B. Displayeinheiten) implementiert werden.

Zusammenfassend kann es erlaubt werden, dass unter Verwendung der vorstehend beschriebenen Metallkomplexe als Emittermaterial mit hoher Lichtausbeute, insbesondere im roten Spektralbereich, und gutem Kontrast Bilder erzeugbar sind, wobei ferner ein energieeffizienter Betrieb möglich ist.

Hinsichtlich weiterer Vorteile und technischer Merkmale des Verfahrens wird auf die Beschreibung der Paracyclophan-Carben-Verbindung wie auch ihrer Verwendung, des Metallkomplexes, seiner Verwendung als Emittermaterial in einer Emitterschicht, der Emitterschicht, der organischen Leuchtdiode, der Figuren wie auch der Beschreibung der Figuren verwiesen.

Nachfolgend wird die Erfindung unter Bezugnahme auf die anliegenden Zeichnungen und Beispiele exemplarisch erläutert, wobei die nachfolgend dargestellten Merkmale sowohl jeweils einzeln als auch in Kombination einen Aspekt der Erfindung darstellen können, und wobei die Erfindung nicht auf die folgende Zeichnung, die folgende Beschreibung und das folgende Ausführungsbeispiel beschränkt ist.

Es zeigen:
Fig. 1 eine schematische Darstellung eines Aufbaus einer OLED in einer Ausgestaltung gemäß der vorliegenden Erfindung;
Fig. 2 eine schematische Darstellung eines Aufbaus einer OLED in einer weiteren Ausgestaltung gemäß der vorliegenden Erfindung;
Fig. 3 ein Reaktionsschema zur Herstellung der erfindungsgemäßen Paracyclophan-Carben-Verbindung;
Fig. 4 ein ¹H-NMR-Spektrum der Verbindung 6;
Fig. 5 ein ¹³C-NMR-Spektrum der Verbindung 6;
Fig. 6 ein ¹H-NMR-Spektrum der Verbindung 7;
Fig. 7 ein ¹³C-NMR-Spektrum der Verbindung 7;
Fig. 8 ein ¹H-NMR-Spektrum der Verbindung 9;
Fig. 9 ein ¹³C-NMR-Spektrum der Verbindung 9;
Fig. 10ein ¹H-NMR-Spektrum der Verbindung 10;
Fig. 11ein ¹³C-NMR-Spektrum der Verbindung 10;
Fig. 12ein ¹H-NMR-Spektrum der Verbindung 11;
Fig. 13 ein ¹³C-NMR-Spektrum der Verbindung 11;
Fig. 14ein ¹H-NMR-Spektrum der Verbindung 12;
Fig. 15ein ¹³C-NMR-Spektrum der Verbindung 12;
Fig. 16 normierte Anregungs- und Emissionsspektren der Verbindungen 6, 7 und 8;
Fig. 17 normierte Anregungs- und Emissionsspektren der Verbindungen 9, 10,11 und 12;
Fig. 18 normierte Anregungs- und Emissionsspektren der Verbindungen 9, 10, 11 und 12;
Fig. 19ein EL-Spektrum einer beispielhaften OLED; und
Fig. 20 die Strom-Spannungskennlinie bei positiver Spannung einer beispielhaften OLED.

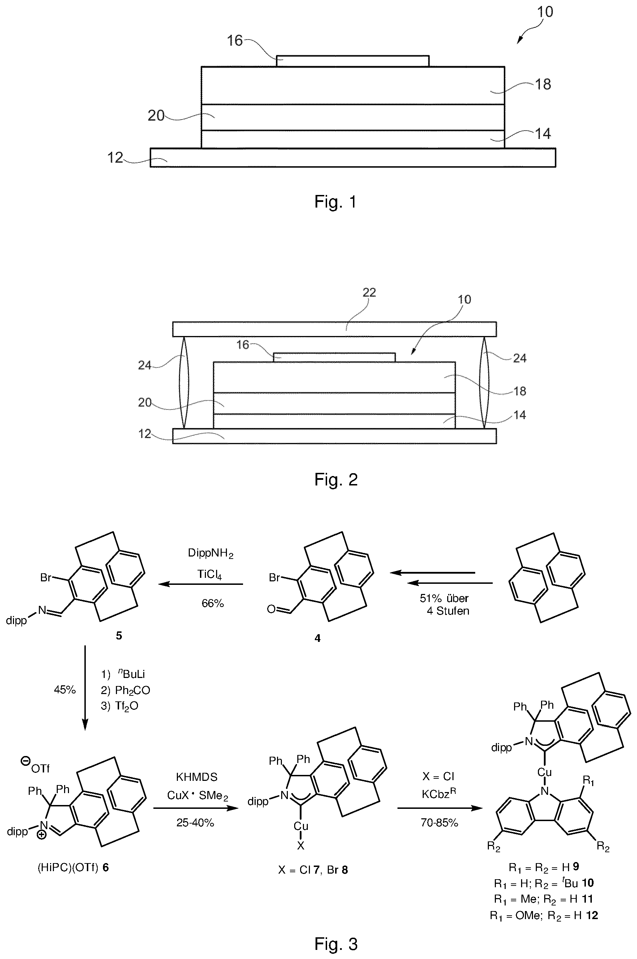

In der Figur 1 ist eine Ausgestaltung eines Schichtaufbaus 10 für eine OLED gemäß einer Ausgestaltung der vorliegenden Erfindung gezeigt. Der Schichtaufbau 10 umfasst eine Anode 14, eine Kathode 16 und eine Emitterschicht 18. Die Anode 14 kann ein Material mit moderater Austrittsarbeit, wie etwa ITO, aufweisen, beispielsweise daraus bestehen, und die Kathode 16 kann ein Material niedriger Austrittsarbeit, wie etwa Calcium, aufweisen beziehungsweise daraus bestehen. Die Emitterschicht 18 umfasst einen Metallkomplex wie nachfolgend beschrieben.

Ferner ist eine Schicht 20 gezeigt, welche eine Lochleitungsschicht darstellt. Diese kann beispielsweise durch das lochleitende Polymer Poly(3,4-ethylenedioxythiophen) Polystyrensulfonat (PEDOT:PSS) realisiert werden.

Grundsätzlich können noch weitere Schichten vorliegen, ohne den Rahmen der Erfindung zu verlassen. So können weiterhin beispielsweise eine Lochtransportschicht zwischen Emitterschicht 18 und Lochleitungsschicht 20 vorgesehen sein, und/oder zwischen Emitterschicht 18 und Kathode 16 kann eine Elektronentransportschicht vorliegen. Grundsätzlich können die Transportschichten ihrerseits auch als Exzitonen- und Ladungsträgerblockierschichten fungieren, oder aufgrund der Energetik mehrere Funktionen in einem erfüllen.

In der Figur 2 ist ferner noch eine Deckschicht 22, wie etwa ein Deckglas, gezeigt, welches den Schichtaufbau 10 nach oben abgrenzt, wobei zwischen der Deckschicht 22 und dem Substrat 12 eine Versiegelung 24 gezeigt ist, die etwa aus einem Klebstoff, wie etwa einem Epoxykleber, ausgebildet sein kann. Durch das Substrat 12, die Deckschicht 22 und die Versiegelung kann der Schichtaufbau 10 somit verkapselt und dadurch vor äußeren Einflüssen geschützt sein. Dadurch kann beispielsweise die Degradation des Calciums und der Organik minimiert werden. In der Verkapselung kann beispielsweise ein Schutzgas, wie etwa Stickstoff, vorliegen.

Gemeinsam ist den Ausgestaltungen der Figuren 1 und 2, dass die Emitterschicht 18 einen definierten Kupferkomplex als Emittermaterial aufweist. Dabei ist es vorgesehen, dass der Metallkomplex der folgenden Struktur (III) entspricht:

L¹Mre[L²][L³]ₘ (III),

wobei L¹ ein Paracyclophan-Carben-Ligand nach einem der Ansprüche 1 bis 8 ist, wobei L² und L³ gleich oder unterschiedlich sind und unabhängig voneinander ausgewählt sind aus NR²³₂, NR²⁴₃, PR²⁵₂, PR²⁶₃, OR²⁷₂, OR²⁸, SR²⁹, SR³⁰₂, SR³¹, SeR³²₂, SeR³⁴, TeR³⁵₂, TeR³⁶, Silyl, Aryl, Heteroaryl, Alkenyl, Alkinyl, Isonitril, Cyclopentadienyl, Halogen, wobei m eine Zahl ist ausgewählt aus 0, 1 oder 2 mit der Maßgabe, dass wenigstens ein m ungleich null ist, wobei
R²³, R²⁴, R²⁵, R²⁶, R²⁷, R²⁸, R²⁹, R³⁰, R³¹, R³², R³³, R³⁴, R³⁵, R³⁶ gleich oder unterschiedlich sind und unabhängig voneinander ausgewählt sind aus Aryl, Heteroaryl, Alkenyl, Alkinyl, Alkyl, Perfluoralkyl, Perfluoraryl; und wobei
Me ein Metall ist, das ausgewählt ist aus Kupfer, Silber und Gold.

Beispielhaft kann es vorgesehen sein, dass der Metallkomplex ausgewählt ist aus den Strukturen (IV), (V), (VI), (VII), (VIII) und (IX) wie nachfolgend gezeigt:

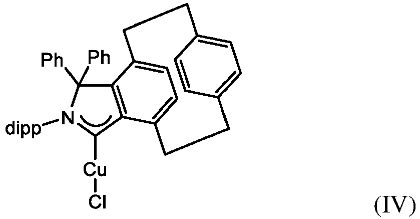

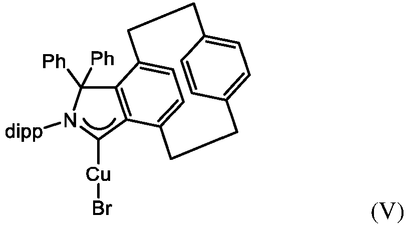

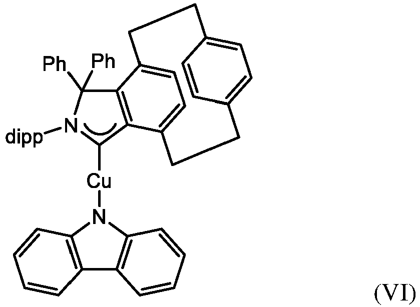

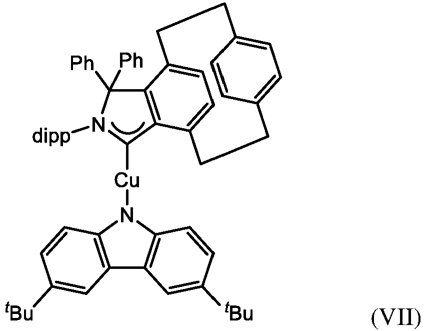

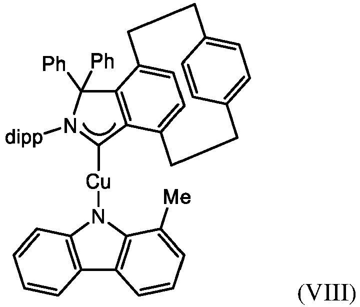

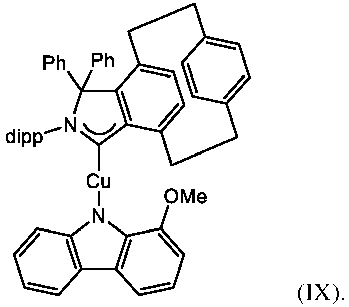

Im Folgenden werden Synthesebeispiele für vorstehend definuerte Emittermaterialien gezeigt, die in der Emitterschicht 18 vorliegen. Dabei wird zunächst auf die Synthese der Liganden eingegangen, also insbesondere des Liganden L¹. Anschließend wird die Synthese verschiedener Metallkomplexe beschrieben.

Dabei entspricht, wie nachfolgend gezeigt, die Struktur (II) der Verbindung 6, die Struktur (IV) der Verbindung 7, die Struktur (VI) der Verbindung 9, die Struktur (VII) der Verbindung 10, die Struktur (VIII) der Verbindung 11, die Struktur (IX) der Verbindung 12, die Struktur (IV) der Verbindung 7, die Struktur (IV) der Verbindung 7.

### Syntheseeispiele für den von dem Kupferkomplex enthaltenen Liganden

Eine Synthese des Paracyclophan-Carben-Liganden beziehungsweise der Paracyclophan-Carben-Verbindung kann ablaufen gemäß dem Schema 1, welches als Figur 3 gezeigt ist.

Im Detail verläuft die Synthese wie folgt:

### rac-4-Formyl[2.2]paracyclophan (Verbindung 1)

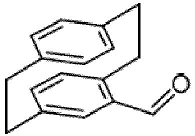

In einem 500 ml Schlenkkolben wurden 2.00 g (9.60 mmol, 1.0 Äq.) -[2.2]Paracyclophan in 150 ml DCM auf 0°C gekühlt. Es wurden 0.90 ml (10 mmol, 1.05 Äq.) Titan(IV)-chlorid und 2.1 ml (19 mmol, 2 Äq.) Dichlormethoxymethan vorsichtig zugegeben und über Nacht gerührt. Die schwarze Reaktionslösung wurde auf 200 ml Eis gegeben. Die türkise Suspension wurde für 2 h gerührt. Die farblose organische Phase wird abgetrennt und die farblose wässrige Phase zweifach mit 50 ml DCM extrahiert. Die vereinigte organische Phase wurde mit Na₂SO₄ getrocknet, filtriert und alle flüchtigen Bestandteile unter verminderten Druck entfernt. Das Produkt wurde nach waschen mit Pentan als farbloser Feststoff. (2.10 g, 8.89 mmol, 93%) erhalten.

Die spektroskopischen Daten sind literaturbekannt.

### rac-4-Formyl[2.2]paracyclophan-O-methylaldoxime (Verbindung 3)

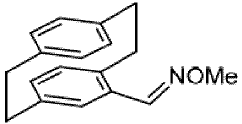

In einem 100 ml Schlenkkolben wurden 424 mg (5.08 mmol, 1.2 Äq.) Methoxyaminhydrochlorid, 0.67 ml (8.4 mmol, 2 Äq.) Pyridin und 0.5 g Molsieb (4 Å) in 12 ml DCM für 5 min gerührt. Es wurden 1.00 g (4.23 mmol, 1 Äq.) rac-4-Formyl[2.2]paracyclophan zugegeben und über Nacht gerührt. Das gelbe Reaktiongemisch wurde dreifach mit 20 ml DCM extrahiert und über Silica filtriert und alle flüchtigen Bestandteile unter verminderten Druck entfernt. Das Produkt wurde durch Säulenchromatografie (Silica; Pentan:Ethylacetat 100:0 → 50:1) als farblosen Feststoff 660 mg, 2.49 mmol, 59%) erhalten.

Die spektroskopischen Daten sind literaturbekannt

### rac-4-Bromo-5-formy[2.2]paracyclophan-O-methylaldoxime (Verbindung xx)

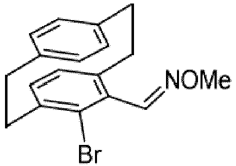

In einem 100 ml Young-Rohr wurden 200 mg (754 µmol, 1 Äq.) *rac-4-*Formyl[2.2]paracyclophan-O-methylaldoxime, 34 mg (0.15 mmol, 0.2 Äq.) Palladium(II)-acetat, 33 mg (0.15 mmol, 0.2 Äq.) Silber(I)-trifluoroacetat und 161 mg (905 pmol, 1.2 Äq.) N-Bromsuccinimid in 25 ml DCE für 6 h bei 100°C gerührt. Das Reaktionsgemisch wurde auf RT abgekühlt und mit 25 ml DCM und 20 ml dest. Wasser versetzt. Die wässrige Phase wurde zweifach mit 20 ml DCM extrahiert. Die organische Phase wurde mit Magnesiumsulfat getrocknet, über Celite filtriert und alle flüchtigen Bestandteile unter verminderten Druck entfernt. Das Rohprodukt wurde ohne weitere Aufarbeitung weiterverwendet.

Die spektroskopischen Daten sind literaturbekannt.

### rac-4-Bromo-5-forrnyl[2.2]paracyclophan (Verbindung 4)

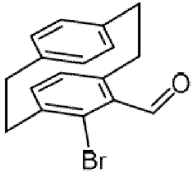

In einem 10 ml Mikrowellenvial wurden 160 mg (465 µmol, 1 Äq.) rac-4-Bromo-5-formyl[2.2]paracyclophan-O-methylaldoxime, 180 mg (946 µmol, 2.0 Äq.) *para-*Toluolsulfonsäure-monohydrat und 0.35 ml (4.7 mmol, 10 Äq., 37% in Wasser) Formaldehyd in 5 ml THF und 1 ml Wasser bei 70 W (120°C) für 4 h mit Mikrowellen bestrahlt. Das Reaktionsgemisch wurde auf RT abgekühlt und alle flüchtigen Bestandteile unter verminderten Druck entfernt. Das Produkt wurde durch Säulenchromatografie (Silica; Cyclohexan:Ethylacetat 100:0 → 50:1) als farblosen Feststoff 140 mg, 0.444 mmol, 96%) erhalten.

Die spektroskopischen Daten sind literaturbekannt

### rac-4-Bromo-5-formyl[2.2]paracyclophan-N-(2,6-diisopropylphenyl)methanimin (Verbindung 5)

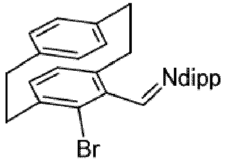

Zu einer Lösung aus 100 mg (317 pmol, 1.0 Äq.) rac-4-Bromo-5-formyl[2.2]paracyclophan und 0.60 mg (0.34 mmol, 1.1 Äq.) N-(2,6-diisopropylphenyl-amin in 1 ml Toluol wurden 0.07 ml (0.6 mmol, 2 Äq.) Titan(IV)-chlorid bei 0°C vorsichtig zugegeben und für 2 h zum Reflux erhitzt. Das orange Reaktionsgemisch wurde in 3 ml Isopropanol gegeben. Es wurden 3 ml dest. Wasser und 4 ml Diethylether zugegeben. Die wässrige Phase wurde zweimal mit 5 ml Diethylether extrahiert. Die vereinigte organische Phase wurde mit Na₂SO₄ getrocknet, dekantiert und alle flüchtigen Bestandteile unter verminderten Druck entfernt. Das Produkt wurde durch Säulenchromatografie (Silica; Cyclohexan:Ethylacetat 100:0 → 50:1) als gelbes Öl 100 mg, 0.211 mmol, 66%) erhalten.

Die ¹H-NMR-Daten sind wie folgt: (500 MHz, CDCl₃) *δ* 8.26 (s, 1H), 7.21 (m, 2H), 7.15 m, 1H), 6.98 (m, 1H), 6.67 (m, 2H), 6.58 (m, 3H), 4.26 (m, 1H), 3.56 (m, 1H), 3.19 (m, 5H), 3.10 (m, 1H), 2.93 (m, 2H), 1.28 (d, *J =* 6.8 Hz, 6H), 1.24 (d, *J* = 6.9 Hz, 6H).

### (HiPC)(OTf) (Verbindung 6)

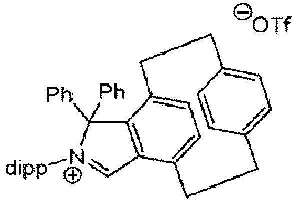

Bei -80°C wurde zu einer Lösung aus 5.40 g (11.4 mmol, 1.0 Äq.) rac-4-Bromo-5-formyl[2.2]paracyclophan-N-(2,6-diisopropylphenyl)methanimin in 15 ml Diethylether langsam 4.8 ml (2.5 M, 12 mmol, 1.05 Äq.) *ⁿ*Butyllithium zugegeben. Die rote Lösung wurde für 1 h bei -80°C gerührt. Es wurde bei -80°C eine Lösung aus 2.20 g (12.1 mmol, 1.05 Äq.) Benzophenon in 10 ml Et₂O zugegeben, wobei sich die Lösung über grün nach violett färbte. Es wurden weitere 30 min bei -80°C und 30 min bei RT gerührt. Zur gelborangen Lösung wurde bei -80°C 2.0 ml (12 mmol, 1.05 Äq.) Trifluormethansulfonsäureanhydrid zugegeben und über Nacht auf RT erwärmt. Der ausgefallene gelbe Feststoff wird abfiltriert und dreifach mit 5 ml Diethylether gewaschen. Das Produkt wurde durch Sublimation von Diethylether in eine THF-Lösung als gelbe Kristalle erhalten (3.60 g, 5.07 mmol, 45%).

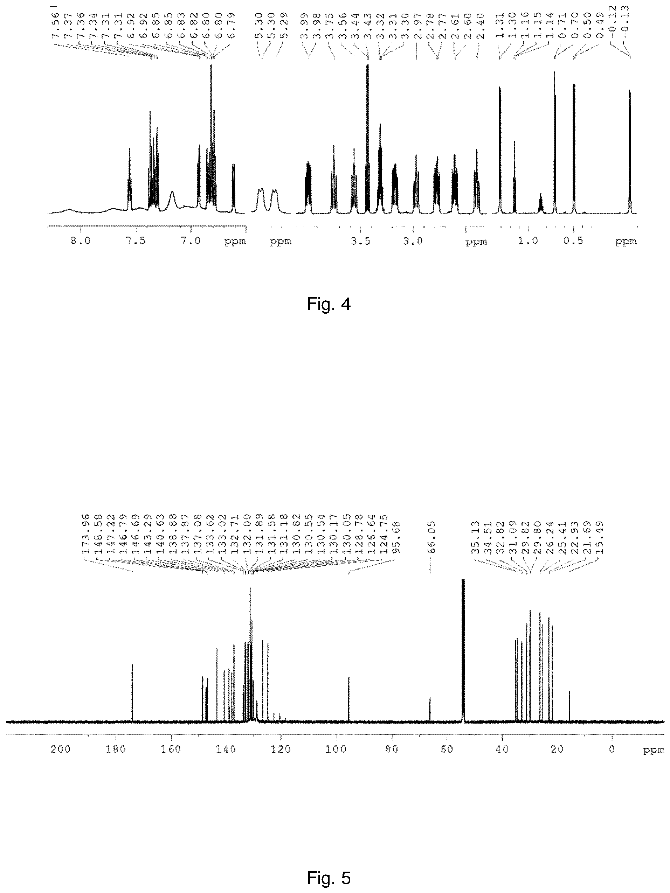

Die spektroskopischen Daten sind wie folgt und in den Figuren 4 (¹H-NMR) und 5 (¹³C-NMR) gezeigt:
¹H-NMR (600 MHz, CD₂Cl₂) *δ* 9.65 (s, 1H), 8.10 (bs, 1H), 7.71 (bs, 1H), 7.56 (m, 1H), 7.45 (bs, 1H), 7.37 (m, 1H), 7.33 (m, 1H), 7.30 (m, 1H), 7.17 (bs, 1H), 7.05 (bs, 1H), 6.93 (m, 1H), 6.85 (m, 1H), 6.82 (m, 1H), 6.80 (m, 1H), 6.78 (m, 1H), 6.61 (m, 1H), 5.29 (m, 1H), 3.99 (m, 1H), 3.75 (m, 1H), 3.56 (m, 1H), 3.31 (sept, *J* = 6.7 Hz, 1H), 3.17 (m, 1H), 2.97 (m, 1H), 2.78 (m, 1H), 2.60 (m, 1H), 2.40 (m, 1H), 1.31 (d, *J =* 6.7 Hz, 3H), 0.86 (sept., *J =* 6.7 Hz, 1H), 0.70 (d, *J* = 6.8 Hz, 3H), 0.49 (d, *J =* 6.7 Hz, 3H), -0.12 (d, *J* = 6.6 Hz, 3H).

¹³C-NMR (151 MHz, CD₂Cl₂) *δ* 174.0 (s), 148.6 (s), 147.2 (s), 146.8 (s), 146.7 (s), 143.3 (s), 140.6 (s), 138.9 (s), 137.9 (s), 137.1 (s), 133.6 (s), 133.0 (s), 132.7 (s), 132.0 (s), 131.9 (s), 131.6 (s), 131.2 (s), 130.8 (s), 130.54 (d, *J* = 1.5 Hz), 130.2 (s), 130.1 (s), 128.8 (s), 126.6 (s), 124.8 (s), 95.7 (s), 35.1 (s), 34.5 (s), 32.8 (s), 31.1 (s), 29.8 (s), 26.2 (s), 25.4 (s), 22.9 (s), 21.7 (s).

¹⁵N-NMR (60.8 MHz, CD₂Cl₂) *δ* -163.4.

¹⁹F-NMR (565 MHz, THF) *δ* -78.55.

Elementaranalyse: Berechnet: C, 72.76; H, 5.96; N, 1.97; Gemessen: C, 72.8; H, 6.2; N, 1.9.

### Synthesebeispiele verschiedener Kupferkomplexe, welche als Emittermaterial dienen können

### [CuCl(iPC)] (Verbindung 7)

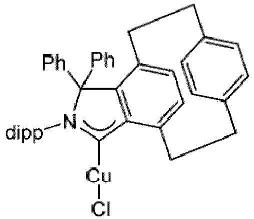

Bei -85°C wurde zu einer Suspension aus 300 mg (423 pmol, 1.0 Äq.) [iPCH](OTf) und 70 mg Kupfer(I)-chlorid · Dimethylsulfid (0.44 mmol, 1.05 Äq.) in 20 ml THF langsam eine Lösung aus 69 mg (0.43 mmol, 1. Äq.) KHMDS in 2 ml THF zugegeben. Die gelborange Suspension wurde über Nacht auf RT erwärmt. Alle flüchtigen Bestandteile der gelbgrünen Suspension wurden unter verminderten Druck entfernt. Das Rohprodukt wurde nach Extraktion mit DCM und Filtration über basischem Aluminiumoxid durch Zugabe von Pentan gefällt und zweifach mit 2 ml Pentan gewaschen. Das Produkt wurde durch Sublimation von Pentan in eine THF/Cyclohexan-Lösung als 1:1-Addukt mit THF als orange Kristalle erhalten (112 mg, 170 pmol, 40%).

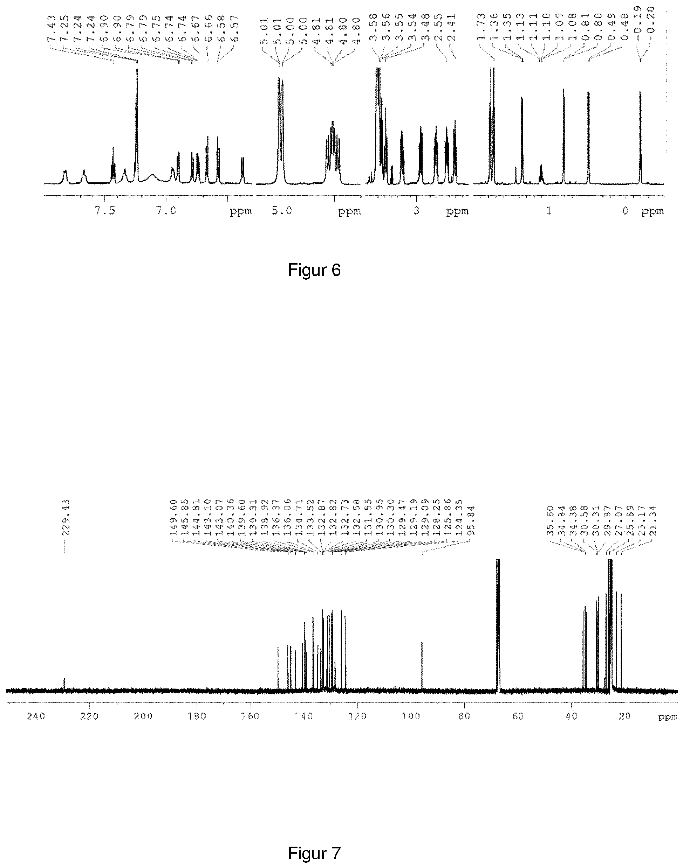

Die spektroskopischen Daten sind wie folgt und in den Figuren 6 (¹H-NMR) und 7 (¹³C-NMR) gezeigt:
¹H-NMR (600 MHz, THF) *δ* 7.82 (m, 1H), 7.67 (m, 1H), 7.43 (m, 1H), 7.34 (m, 1H), 7.24 (m, 3H), 7.11 (bs, 2H), 6.95 (m, 1H), 6.90 (m, 1H), 6.79 (m, 1H), 6.74 (m, 1H), 6.67 (m, 1H), 6.58 (m, 1H), 6.38 (m, 1H), 5.00 (m, 1H), 4.81 (m, 1H), 3.54 (sept., *J* = 6.7 Hz, 1H), 3.48 (m, 1H), 3.23 (m, 1H), 2.94 (m, 1H), 2.71 (m, 1H), 2.54 (m, 1H), 2.41 (m, 1H), 1.35 (d, *J =* 6.8 Hz, 3H), 1.10 (sept., *J* = 6.8 Hz, 1H), 0.81 (d, *J =* 6.7 Hz, 3H), 0.49 (d, *J =* 6.7 Hz, 3H), -0.20 (d, *J =* 6.7 Hz, 3H).

¹³C-NMR (151 MHz, THF) *δ* 229.4 (s), 149.6 (s), 145.9 (s), 144.8 (s), 143.1 (s), 143.1 (s), 140.4 (s), 139.6 (s), 139.3 (s), 138.9 (s), 136.4 (s), 136.1 (s), 134.7 (s), 133.5 (s), 132.9 (s), 132.8 (s), 132.7 (s), 132.6 (s), 131.6 (s), 131.0 (s), 130.3 (s), 129.5 (s), 129.2 (s), 129.1 (s), 128.3 (s), 125.9 (s), 124.4 (s), 95.8 (s), 35.6 (s), 34.8 (s), 34.4 (s), 30.6 (s), 30.3 (s), 29.9 (s), 27.1 (s), 23.2 (s), 21.3 (s).

### Elementar Analyse: Berechnet: C, 75.17; H, 6.97; N, 1.83; Gemessen: C, 75.1; H, 6.9; N, 1.9. [CuBr(iPC)] (Verbindung 8)

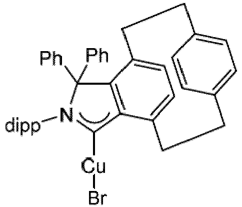

Bei -85°C wurde zu einer Suspension aus 300 mg (423 pmol, 1.0 Äq.) [iPCH](OTf) und 87 mg Kupfer(I)-bromid · Dimethylsulfid (0.42 mmol, 1.05 Äq.) in 20 ml THF langsam eine Lösung aus 69 mg (0.43 mmol, 1. Äq.) KHMDS in 2 ml THF zugegeben. Die gelborange Suspension wurde über Nacht auf RT erwärmt. Alle flüchtigen Bestandteile der gelborangen Suspension wurden unter verminderten Druck entfernt. Das Rohprodukt wurde nach Extraktion mit DCM und Filtration über basischem Aluminiumoxid durch Zugabe von Pentan gefällt und zweifach mit 2 ml Pentan gewaschen. Das Produkt wurde durch Sublimation von Pentan in eine THF/Cyclohexan-Lösung als orange Kristalle erhalten (21 mg, 30 µmol, 7%).

Die spektroskopischen Daten sind wie folgt:
¹H-NMR (600 MHz, THF) *δ* 7.82 (m, 1H), 7.67 (m, 1H), 7.43 (m, 1H), 7.34 (m, 1H), 7.24 (m, 3H), 7.11 (bs, 2H), 6.95 (m, 1H), 6.90 (m, 1H), 6.79 (m, 1H), 6.74 (m, 1H), 6.67 (m, 1H), 6.58 (m, 1H), 6.38 (m, 1H), 5.00 (m, 1H), 4.81 (m, 1H), 3.54 (sept., *J* = 6.7 Hz, 1H), 3.48 (m, 1H), 3.23 (m, 1H), 2.94 (m, 1H), 2.71 (m, 1H), 2.54 (m, 1H), 2.41 (m, 1H), 1.35 (d, *J =* 6.8 Hz, 3H), 1.10 (sept., *J* = 6.8 Hz, 1H), 0.81 (d, *J =* 6.7 Hz, 3H), 0.49 (d, *J =* 6.7 Hz, 3H), -0.20 (d, *J =* 6.7 Hz, 3H).

### Elementar Analyse: Berechnet: C, 71.73; H, 5.88; N, 1.99; Gemessen: C, 71.4; H, 6.0; N, 1.8. [Cu(Cbz)(iPC)] (Verbindung 9)

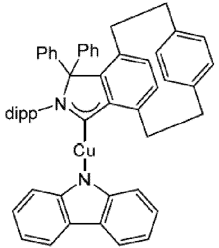

In einem 20 ml-Schraubdeckelgefäß wurden 16 mg (78 pmol, 1.03 Äq.) KCbz zu einer Lösung aus 50 mg (76 pmol, 1.0 Äq.) [CuCl(iPC)] in 4 ml THF gegeben und über Nacht gerührt. Die tiefrote Suspension wurde mit 2 ml Diethylether versetzt und über basischem Aluminiumoxid filtriert und unter verminderten Druck auf ein Viertel des Volumens eingeengt. Das Produkt wurde durch Sublimation einer Mischung aus Cyclohexan und Pentan in die Lösung als 2:3-Addukt mit THF als gelbe Kristalle (45 mg, 57 pmol, 75%) erhalten.

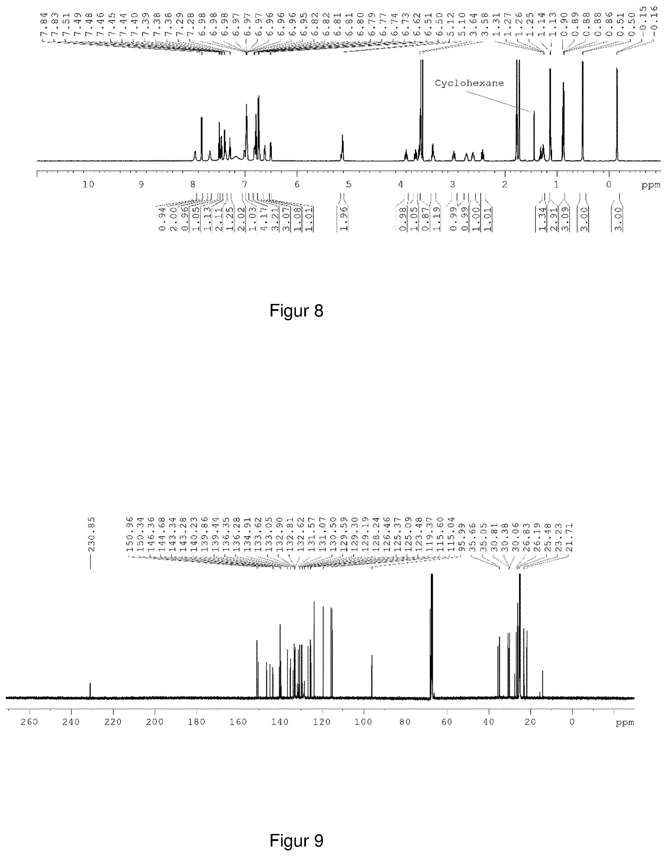

Die spektroskopischen Daten sind wie folgt und in den Figuren 8 (¹H-NMR) und 9 (¹³C-NMR) gezeigt:
¹H-NMR (600 MHz, THF) *δ* 7.96 m, 1H), 7.83 m, 2H), 7.67 (m, 1H), 7.49 (m, 1H), 7.45 (m, 1H), 7.38 (m, 2H), 7.29 (m, 1H), 7.17 (bs, 21H), 7.01 (m, 1H), 6.97 (m, 4H), 6.80 (m, 3H), 6.73 (m, 3H), 6.62 (m, 1H), 6.50 (m, 1H), 5.13 (m, 2H), 3.90 (m, 1H), 3.72 (m, 1H), 3.64 (sept., *J* = 6.8 Hz, 1H), 3.37 (m, 1H), 2.98 (m, 1H), 2.74 (m, 1H), 2.61 (m, 1H), 2.43 (m, 1H), 1.26 (m, 1H), 1.13 (d, *J =* 6.7 Hz, 3H), 0.87 (d, *J =* 6.8 Hz, 3H), 0.51 (d, *J =* 6.7 Hz, 3H), - 0.16 (d, *J* = 6.7 Hz, 3H).

¹³C-NMR (151 MHz, THF) *δ* 230.9 (s), 151.0 (s), 150.3 (s), 146.4 (s), 144.7 (s), 143.3 (s), 143.3 (s), 140.2 (s), 139.9 (s), 139.4 (s), 136.4 (s), 136.3 (s), 134.9 (s), 133.6 (s), 133.1 (s), 132.9 (s), 132.8 (s), 132.6 (s), 131.6 (s), 131.1 (s), 130.5 (s), 129.6 (s), 129.3 (s), 129.2 (s), 128.2 (s), 126.5 (s), 125.4 (s), 125.1 (s), 123.5 (s), 119.4 (s), 115.6 (s), 115.0 (s), 96.0 (s), 35.7 (s), 35.1 (s), 30.8 (s), 30.4 (s), 30.1 (s), 26.8 (s), 26.2 (s), 25.5 (s), 23.2 (s), 21.7 (s). Elementaranalyse: Berechnet: C, 80.28; H, 6.85; N, 3.12; Gemessen: C, 79.9; H, 6.9; N, 3.3.

### [Cu(Cbz^{tBu})(ipC)l (Verbindung 10)

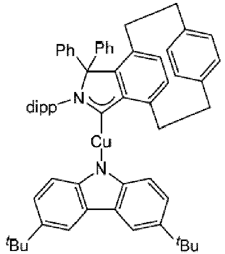

In einem 20 ml-Schraubdeckelgefäß wurden 28 mg (77 µmol, 1 Äq.) [KCbz^{tBu} · (Et2O)_{2/3}] zu einer Lösung aus 50 mg (76 pmol, 1.0 Äq.) [CuCl(iPC)] in 4 ml THF gegeben und über Nacht gerührt. Die tiefrote Suspension wurde mit 2 ml Diethylether versetzt und über basischem Aluminiumoxid filtriert und unter verminderten Druck auf ein Viertel des Volumens eingeengt. Das Produkt wurde durch Sublimation einer Mischung aus Cyclohexan und Pentan in die Lösung als 4:5-Addukt mit THF als gelbe Kristalle (51 mg, 57 pmol, 75%) erhalten.

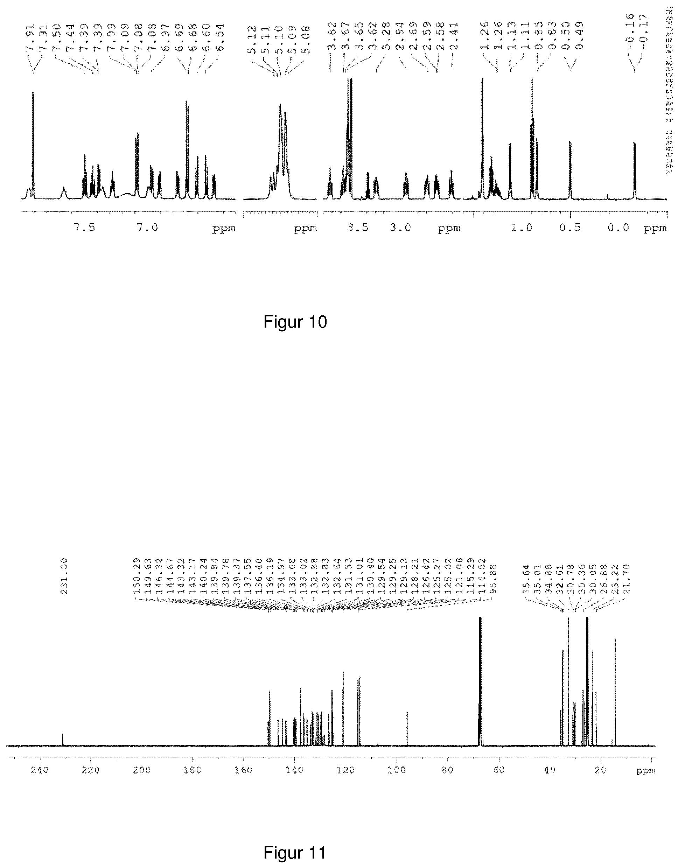

Die spektroskopischen Daten sind wie folgt und in den Figuren 10 (¹H-NMR) und 11 (¹³C-NMR) gezeigt:
¹H-NMR (600 MHz, THF) *δ* 7.94 (m, 1H), 7.91 (m, 2H), 7.66 (m, 1H), 7.50 (m, 1H), 7.44 (m, 1H), 7.39 (m, 1H), 7.36 (m, 1H), 7.28 (m, 1H), 7.16 (bs, 2H), 7.08 (m, 2H), 6.99 (m, 1H), 6.97 (m, 1H), 6.91 (m, 1H), 6.76 (m, 1H), 6.68 (m, 2H), 6.61 (m, 1H), 6.53 (m, 1H), 5.12 (m, 1H), 5.09 (m, 1H), 3.82 (m, 1H), 3.67 (m, 1H), 3.29 (m, 1H), 2.94 (m, 1H), 2.70 (m, 1H), 2.58 (m, 1H), 2.41 (m, 1H), 1.41 (s, 18H), 1.26 (m, 1H), 1.12 (d, *J* = 6.7 Hz, 3H), 0.84 (d, *J*= 6.7 Hz, 3H), 0.50 (d, *J* = 6.7 Hz, 3H), -0.17 (d, *J* = 6.7 Hz, 3H).

¹³C-NMR (151 MHz, THF) *δ* 231.0 (s), 150.3 (s), 149.6 (s), 146.3 (s), 144.7 (s), 143.3 (s), 143.2 (s), 140.2 (s), 139.8 (s), 139.8 (s), 139.4 (s), 137.6 (s), 136.4 (s), 136.2 (s), 135.0 (s), 133.7 (s), 133.0 (s), 132.9 (s), 132.8 (s), 132.6 (s), 131.5 (s), 131.0 (s), 130.4 (s), 129.5 (s), 129.3 (s), 129.1 (s), 128.2 (s), 126.4 (s), 125.3 (s), 125.0 (s), 121.1 (s), 115.3 (s), 114.5 (s), 95.9 (s), 35.6 (s), 35.0 (s), 34.9 (s), 32.6 (s), 30.8 (s), 30.4 (s), 30.1 (s), 26.9 (s), 23.2 (s), 21.7 (s).

Elementaranalyse: Berechnet: 81.13; H, 7.62; N, 2.82; Gemessen: C, 81.0; H, 7.3; N, 3.1.

### [Cu(^{Me}Cbz)(iPC)] (Verbindung 11)

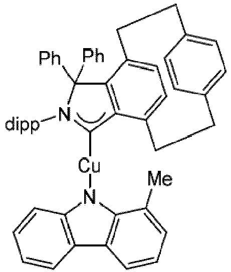

In einem 20 ml-Schraubdeckelgefäß wurden 17 mg (78 µmol, 1 Äq.) K^{Me}Cbz zu einer Lösung aus 50 mg (76 pmol, 1.0 Äq.) [CuCl(iPC)] in 4 ml THF gegeben und über Nacht gerührt. Die tiefrote Suspension wurde mit 2 ml Diethylether versetzt und über basischem Aluminiumoxid filtriert und unter verminderten Druck auf ein Viertel des Volumens eingeengt. Das Produkt wurde durch Sublimation einer Mischung aus Cyclohexan und Pentan in die Lösung als 2:3-Addukt mit THF als gelbe Kristalle (48 mg, 60 pmol, 79%) erhalten.

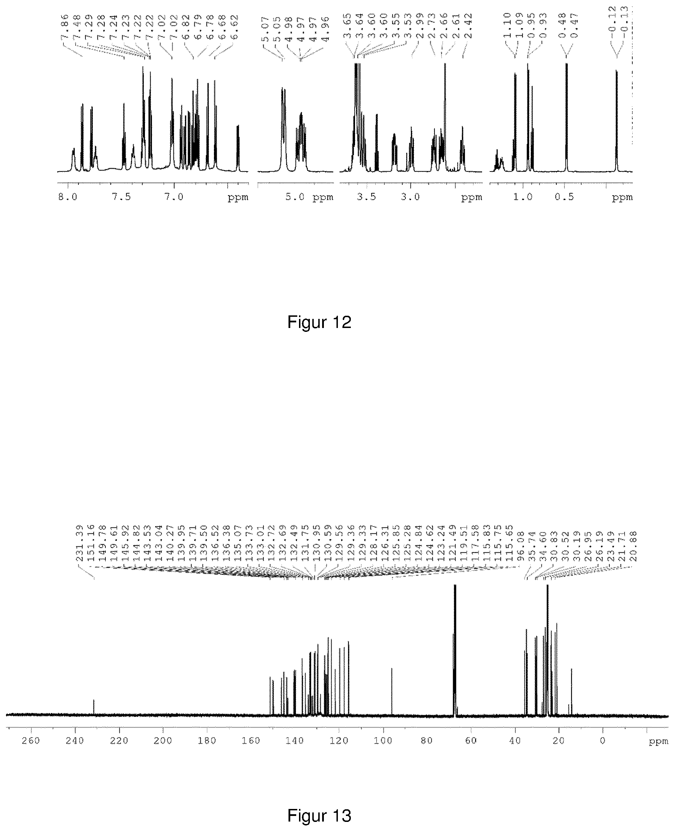

Die spektroskopischen Daten sind wie folgt und in den Figuren 12 (¹H-NMR) und 13 (¹³C-NMR) gezeigt:
¹H-NMR (600 MHz, THF) *δ* 7.95 (m, 1H), 7.87 (m, 1H), 7.78 (m, 1H), 7.74 (m, 1H), 7.60 (bs, 1H), 7.48 (m, 1H), 7.39 (m, 1H), 7.29 (m, 3H), 7.22 (m, 2H), 7.02 (m, 3H), 6.93 (m, 1H), 6.90 (m, 1H), 6.86 (m, 1H), 6.82 (m, 1H), 6.79 (m, 1H), 6.78 (m, 1H), 6.69 (m, 1H), 6.61 (m, 1H), 6.40 (m, 1H), 5.06 (m, 1H), 4.97 (m, 1H), 3.64 (sept., *J* = 6.7 Hz, 1H), 3.60 (m, 1H), 3.53 (m, 1H), 3.18 (m, 1H), 2.99 (m, 1H), 2.74 (m, 1H), 2.65 (m, 1H), 2.61 (s, 3H), 2.42 (m, 1H), 1.25 (m, 1H), 1.09 (d, *J =* 6.8 Hz, 3H), 0.94 (d, *J =* 6.7 Hz, 3H), 0.47 (d, *J =* 6.7 Hz, 3H). ¹³C-NMR (151 MHz, THF) *δ* 231.4 (s), 151.2 (s), 149.8 (s), 149.6 (s), 145.9 (s), 144.8 (s), 143.5 (s), 143.0 (s), 140.3 (s), 140.0 (s), 139.7 (s), 139.5 (s), 136.5 (s), 136.4 (s), 135.1 (s), 133.7 (s), 133.0 (s), 132.7 (s), 132.7 (s), 132.5 (s), 131.8 (s), 131.0 (s), 130.6 (s), 129.6 (s), 129.4 (s), 129.3 (s), 128.2 (s), 126.3 (s), 125.9 (s), 125.3 (s), 124.8 (s), 124.6 (s), 123.2 (s), 121.5 (s), 119.5 (s), 117.6 (s), 115.8 (s), 115.8 (s), 115.7 (s), 96.1 (s), 35.7 (s), 34.6 (s), 30.8 (s), 30.5 (s), 30.2 (s), 27.0 (s), 26.2 (s), 23.5 (s), 21.7 (s), 20.9 (s).

Elementaranalyse: Berechnet: C, 80.36; H, 6.97; N, 3.07; Gemessen: C, 80.4; H, 6.9; N, 3.3.

### [Cu(^{OMe}Cbz)(iPC)] (Verbindung 12)

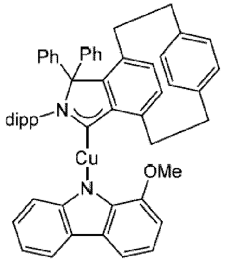

In einem 20 ml-Schraubdeckelgefäß wurden 20 mg (76 µmol, 1 Äq.) [K^{OMe}CBz · (Et2O)_{1/3}] zu einer Lösung aus 50 mg (76 pmol, 1.0 Äq.) [CuCl(iPC)] in 4 ml THF gegeben und über Nacht gerührt. Die tiefrote Suspension wurde mit 2 ml Diethylether versetzt und über basischem Aluminiumoxid filtriert und unter verminderten Druck auf ein Viertel des Volumens eingeengt. Das Produkt wurde durch Sublimation einer Mischung aus Cyclohexan und Pentan in die Lösung als 2:3-Addukt mit THF als gelbe Kristalle (46 mg, 56 pmol, 74%) erhalten.

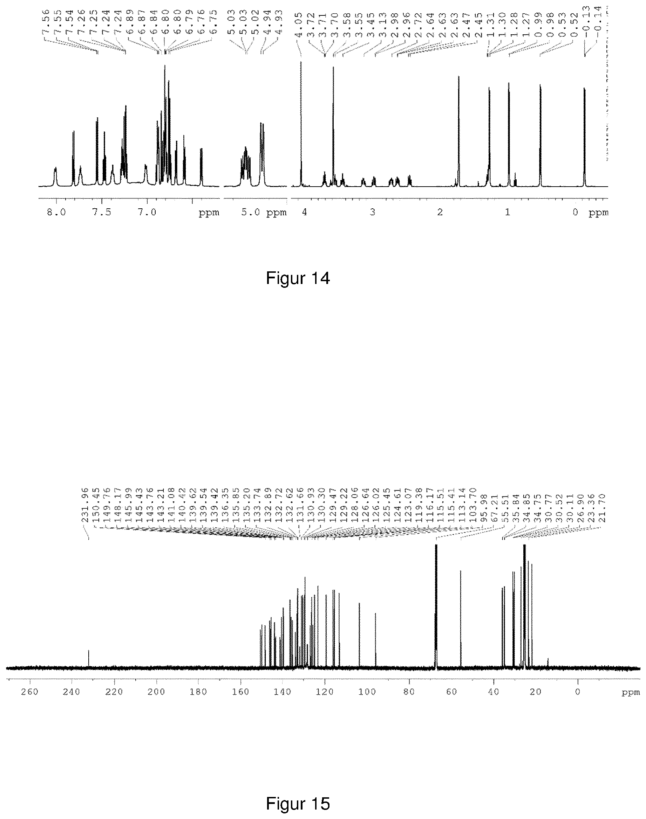

Die spektroskopischen Daten sind wie folgt und in den Figuren 14 (¹H-NMR) und 15 (¹³C-NMR) gezeigt:
¹H-NMR (600 MHz, THF) *δ* 8.01 (m, 1H), 7.81 (m, 1H), 7.73 (m, 1H), 7.55 (m, 1H), 7.47 (m, 1H), 7.38 (m, 1H), 7.28 (m, 1H), 7.26 (m, 1H), 7.24 (d, *J* = 2.8 Hz, 1H), 7.23 (m, 1H), 7.01 (m, 1H), 6.89 (m, 2H), 6.84 (m, 1H), 6.80 (m, 3H), 6.75 (m, 2H), 6.68 (m, 1H), 6.59 (m, 1H), 6.40 (m, 1H), 5.03 (m, 1H), 4.94 (m, 1H), 4.05 (s, 3H), 3.71 (sept., *J* = 6.7 Hz, 1H), 3.55 (m, 1H), 3.45 (m, 1H), 3.14 (m, 1H), 2.98 (m, 1H), 2.73 (m, 1H), 2.63 (m, 1H), 2.45 (m, 1H), 1.30 (sept., J = 6.7 Hz, 1H), 1.28 (d, *J* = 6.8 Hz, 3H), 0.98 (d, *J =* 6.7 Hz, 3H), 0.52 (d, *J =* 6.7 Hz, 3H), -0.13 (d, *J =* 6.7 Hz, 3H).

¹³C-NMR (151 MHz, THF) *δ* 232.0 (s), 150.5 (s), 149.8 (s), 148.2 (s), 146.0 (s), 145.4 (s), 143.8 (s), 143.2 (s), 141.1 (s), 140.4 (s), 139.6 (s), 139.5 (s), 139.4 (s), 136.4 (s), 135.9 (s), 135.2 (s), 133.7 (s), 132.9 (s), 132.7 (s), 132.6 (s), 131.7 (s), 130.9 (s), 130.3 (s), 129.5 (s), 129.2 (s), 128.1 (s), 126.6 (s), 126.0 (s), 125.5 (s), 124.6 (s), 123.1 (s), 119.4 (s), 116.2 (s), 115.5 (s), 115.4 (s), 113.1 (s), 103.7 (s), 96.0 (s), 55.5 (s), 35.8 (s), 34.9 (s), 34.8 (s), 30.8 (s), 30.5 (s), 30.1 (s), 26.9 (s), 23.4 (s), 21.7 (s).

Elementaranalyse: Berechnet: C, 79.47; H, 6.67; N, 3.14; Gemessen: C, 79.4; H, 6.3; N, 3.4
Im Folgenden werden photophysikalische Eigenschaften erfindungsgemäßer Verbindungen gezeigt. UV-Vis-Spektren wurden auf einem Gerät der Firma PerkinElmer, Modell LAMBDA^{™} 265, aufgenommen. Lebenszeitbestimmungen, Aufnahmen von Anregungs- und Emissionsspektren wurden mit einem FLSP920-Spektrometer der Firma Edinburgh Instruments durchgeführt. Die Emissionsstrahlung wurde hierbei jeweils in einem 90°-Winkel zur Anregungsstrahlung aufgezeichnet. Quantenausbeuten wurden mittels einer Ulbricht-Kugel bestimmt. Quantenausbeuten wurden mit einem FLSP920-Spektrometer der Firma Edinburgh Instruments aufgenommen. Alle Messungen wurden in 1 cm Quarzglas-Küvetten durchgeführt.

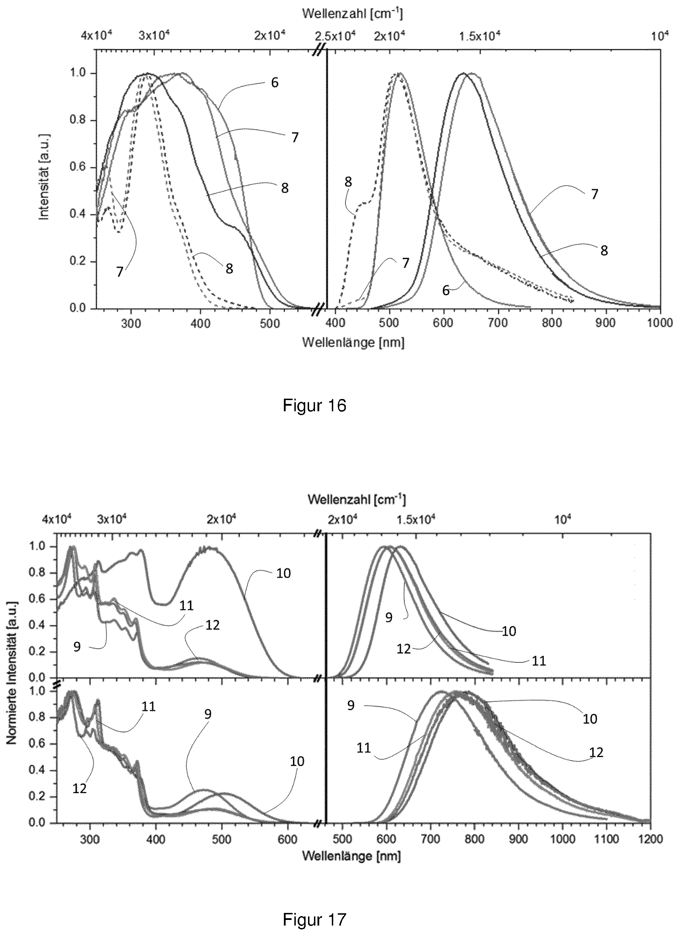

Figur 16 zeigt normierte Anregungs- und Emissionsspektren der Verbindungen 6, 7 und 8, wobei die entsprechenden Kurven mit den Nummern der jeweiligen Verbindungen bezeichnet sind. Dabei sind normierte Anregungs- und Emissionsspektren im gemörserten Festkörper (Linie) oder in PMMA-Matrix (Striche) gezeigt. Bei der Bande in den Emissionsspektren bei 425 nm handelt es sich um Streuung des Lichts, mit welchem die Probe angeregt wurde.

Ausgewählte photophysikalische Daten der Verbindungen 6, 7 und 8 unter Argon bei Raumtemperatur sind ferner in der Tabelle 1 gezeigt.

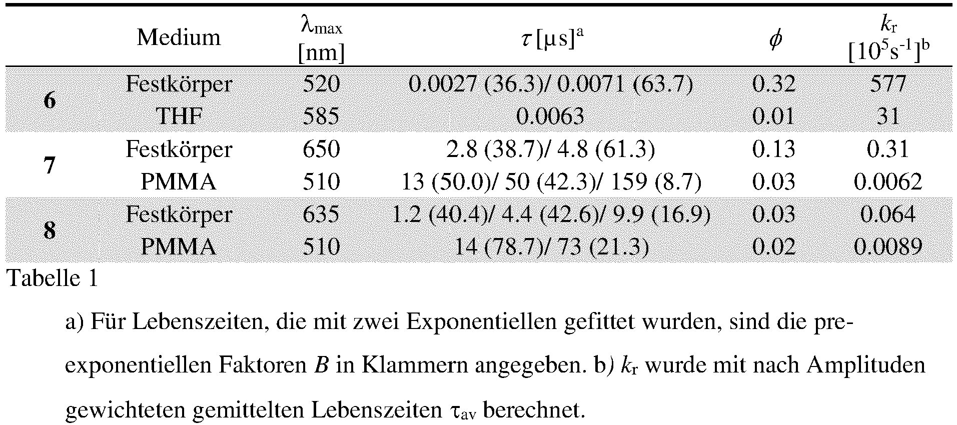

Figur 17 zeigt normierte Anregungs- (links) und Emissionsspektren (rechts) der Verbindungen 9, 10, 11 und 12 in THF-Lösung (unten) oder PMMA-Matrix (oben), wobei die entsprechenden Kurven mit den Nummern der jeweiligen Verbindung bezeichnet sind.

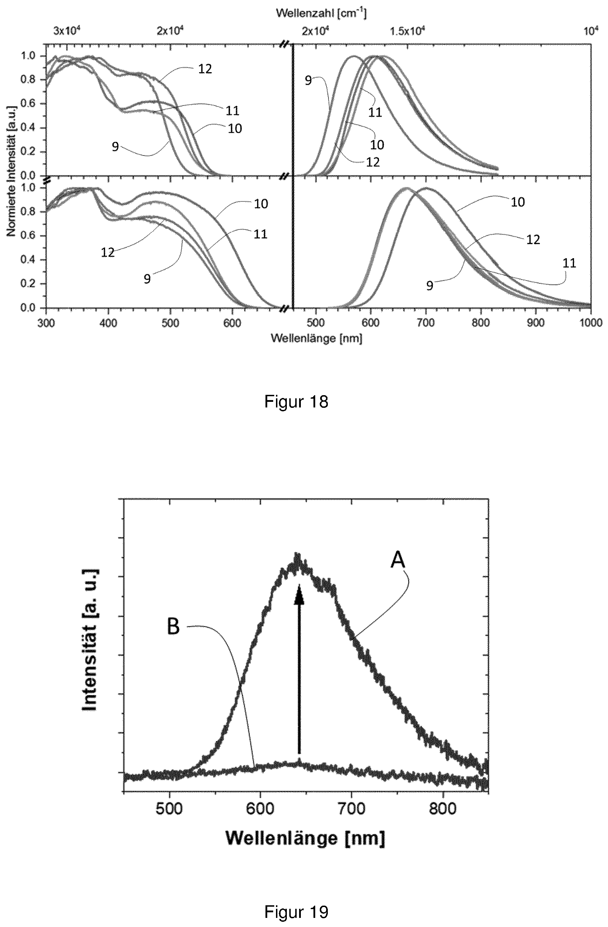

Figur 18 zeigt normierte Anregungs- (links) und Emissionsspektren (rechts) der Verbindungen 9, 10, 11 und 12 in gemörserten (unten) oder mikrokristallinen Zustand (oben), wobei die entsprechenden Kurven mit den Nummern der jeweiligen Verbindung bezeichnet sind.

Ausgewählte photophysikalische Daten Verbindungen 9, 10, 11 und 12 unter Argon bei Raumtemperatur bzw. unter Helium bei 77 K und unter Vakuum bei 6 K sind ferner in der Tabelle 2 gezeigt.

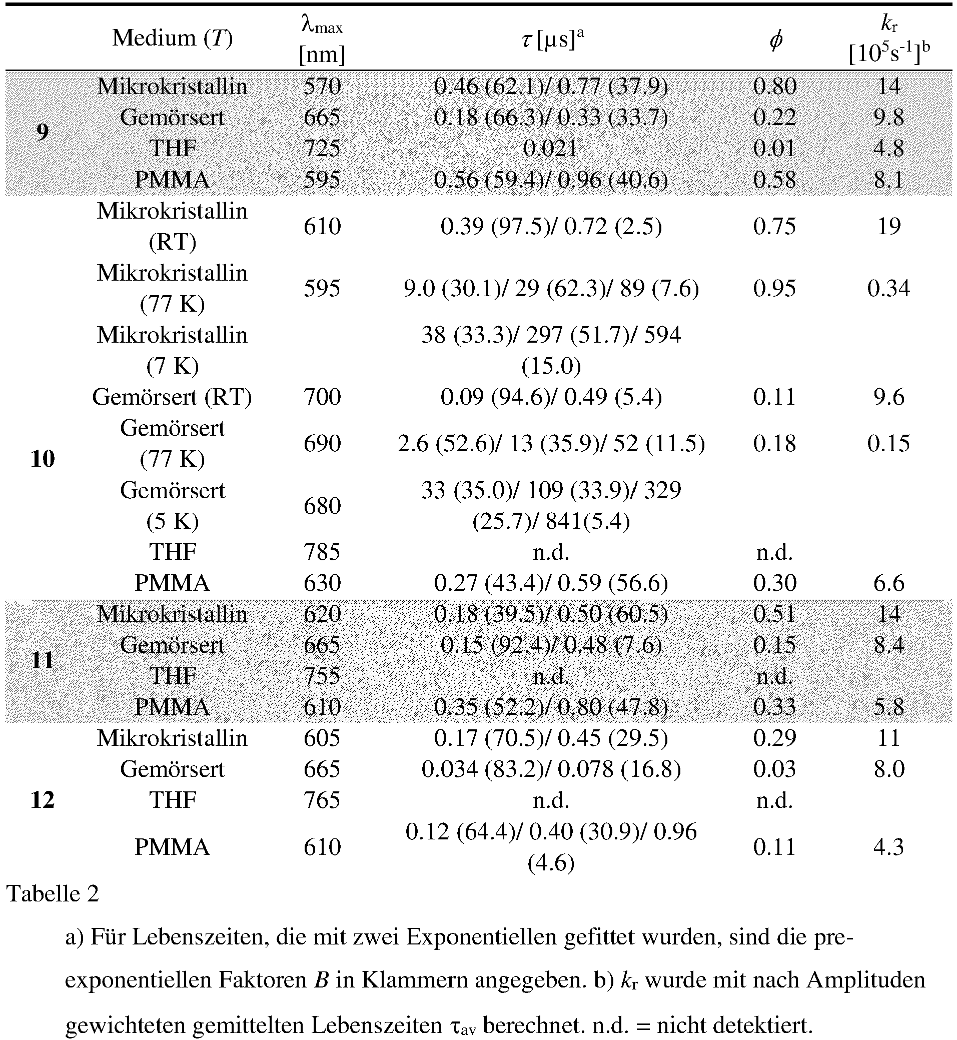

*λ*ₘₐₓ gibt das Maximum der Photolumineszenz (PL) an und ist somit ein wichtiger Parameter für die resultierende im Auge erscheinende Farbe. Die hier gefundenen liegen mit bis zu 785 nm im nahinfraroten, was sie für diverse Lumineszenzanwendungen interessant macht.

Die Quantenausbeute Φ_{PL} ist ein Maß für die Effizienz eines Luminophors. Sie gibt an, welcher Anteil angeregter Zustände strahlend in den Grundzustand übergehen und kann als Verhältnis von emittierten zu absorbierten Photonen aufgefasst werden. Obwohl sich die hier angegebene Φ_{PL} wiederrum auf PL bezieht, ist dieser Wert auch ein interessantes Maß für die Effizienz der Elektrolumineszenz innerhalb eines elektrisch betriebenen Bauteils. Die Lebenszeit *τ* ist ein Maß dafür, wie schnell nach erfolgter Anregung die Luminophore wieder in den Grundzustand zurückkehren (wobei sie bei Φ_{PL} = 1 ein Photon aussenden). *τ* sollte möglichst klein sein um "Effizienz-roll-off" Effekte in OLEDs zu vermeiden. Aus Φ_{PL} und τ lässt sich die strahlenden Ratenkonstante k*_{R}* = Φ_{PL}/τ verrechnen. Mit dieser lassen sich die Komplexe mit anderen Emittern vergleichen. Der höchste hier bestimmte Wert von *k*_{R} = 19x10⁵ s⁻¹ übersteigt dabei bereits viele aktuell eingesetzten Ir- oder Pt-Emittern. Generell existieren kaum Emittermaterialien, die eine so hohe strahlende Ratenkonstante aufweisen.

Im Folgenden werden chiroptische Eigenschaften erfindungsgemäßer Verbindungen beschrieben.

Die Anisotropiefaktoren gabs und glum sind ein Maß wie sehr links- bzw. rechtsdrehende Photonen über die jeweils anderen bei Absorption bzw. Emission dominieren. Dabei gilt: ${g}_{lum} = 2 \frac{Δ I}{I} = \frac{{I}_{L} - {I}_{R}}{\frac{1}{2} \left({I}_{L}+{I}_{R}\right)}$ wobei I_{L} und I_{R} für die Intensität der links- (L) bzw. rechtsdrehende (R) Photonen stehen. Aus der Gleichung ergeben sich die Maxima +2 für reines linksdrehendes bzw. -2 für reines rechtsdrehendes emittiertes Licht. Bei oktaedrischen Übergangsmetallkomplexen (wie Ir(III)) und vergleichbaren bekannten Kupferkomplex liegen diese Werte nur selten über 10⁻³.

Analog wird ${g}_{abs} = \frac{Δϵ}{ϵ} = \frac{{ϵ}_{L} - {ϵ}_{R}}{\frac{1}{2} \left({ϵ}_{L}+{ϵ}_{R}\right)}$ aus den Extinktionskoeffizienten *ε*_{L} und *ε_{R}* für die jeweiligen absorbierten Photonen. *g_{abs}* ist eigentlich für uns weniger wichtig. Dabei wurde für die niederenergetischste Absorption ein Anisotropiefaktoren *g*_{abs} ≈ 2x10⁻³ berechnet. Der *g_{abs}* der energetisch niedrigsten Bande sollte in derselben Größenordnung liegen, wie der *gₗᵤₘ* Wert, da bei einem TADF Mechanismus der Emission dieselben elektronischen Zustände beteiligt sein sollten wie bei der niederenergetischsten Absorption (vgl. Einsteinkoeffizienten). Es wird erfindungsgemäß einen Wert von *gₗᵤₘ* ≈ 2x10⁻³ erwartet. Dieser ist dann durchaus mit denen phosphoreszierender bzw. TADF-Emitter vergleichbar.

Hinsichtlich der Applikation in organischen Leuchtdioden ist folgendes zu sagen.

Durch Integration eines molekularen Emitters in eine organische Leuchtdiode (OLED) ist analog zur optischen Anregung (PL) eine elektrische Anregung des Moleküls möglich, was als Elektrolumineszenz (EL) bezeichnet wird. OLEDs sind insbesondere relevant für Display-/Bildschirmanwendungen. Der beschriebene TADF-Emissions-charakter der erfindungsgemäßen Emitter einhergehend mit hohen Quantenausbeu-ten Φ_{PL} macht diese Materialien besonders interessant für den Einsatz in OLED-Bauteilen, da so theoretisch hohe externe Quanteneffizienzen (EQE) erreicht werden können. Die EQE ist definiert über die Anzahl der Photonen, die pro injizierten Ladungsträger aus der OLED ausgekoppelt werden. Ein wesentlicher Einflussfaktor ist die interne Quanteneffizienz (IQE), welche für phosphoreszente und TADF-Emitter ein Maximum von 100% erreichen kann.

Um den elektrolumineszenten Betrieb der iPC-Emitter zu testen, wurde die Verbindung 10 in eine einfache Proof-Of-Concept-OLED integriert. Die Bauteil-Architektur kann in Verbindung mit Figur 1 beschrieben werden. Indiumzinnoxid (ITO) fungiert als transparente Anode 14 (lochinjizierender Kontakt), PEDOT:PSS als Lochinjektions-/transportmaterial und Calcium/Aluminium als elektroneninjizierender Top-Kontakt. Der Emitter wird über Lösungsprozessierung in ein Matrixmaterial (mCP) eingebettet in einer Menge von 10 Gew.-%, bezogen auf die Schicht. Diese emittierende Schicht (bestehend aus Emitter und Matrix) befindet sich zwischen der PEDOT:PSS-Schicht und dem Top-Kontakt. Bei bipolarer Ladungsträgerinjektion in die emittierende Schicht werden Exzitonen (angeregte Zustände) auf dem Emitter generiert, die strahlend zerfallen können (EL) und in einer Lichtauskopplung über die transparente ITO-Anode resultieren.

Figur 19 zeigt ferner, dass die gemessenen EL-Spektren hinsichtlich der spektralen Lage und Form mit den PL-Spektren übereinstimmen, was zeigt, dass der erfindungsgemäße Emitter gemäß Verbindung 10 in OLED-Bauteilen elektrisch getrieben werden können. Die Stromdichte liegt hierbei in einer OLED-typischen Größenordnung. Die Intensität der EL skaliert wie erwartet mit der Zunahme der Stromdichte. Dabei zeigt die Kurve A eine Stromdichte j von 33 mA/cm² und zeigt die Kurve B eine Stromdichte von 17 mA/cm².

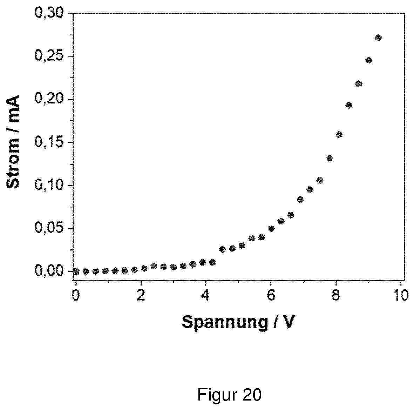

Figur 20 zeigt die Strom-Spannungskennlinie bei positiver Spannung (Durchlassrichtung) und zeigt dabei ein typisches Diodenverhalten. Es wird ersichtlich, dass der Stromfluss in der OLED durch die Injektion über die Kontaktgrenzflächen dominiert wird.

## Patentansprüche

1. Paracyclophan-Carben-Verbindung, **dadurch gekennzeichnet, dass** die Paracyclophan-Carben-Verbindung der folgenden Struktur (I) entspricht: 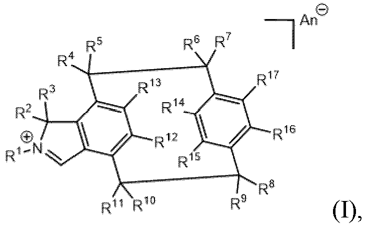 wobei
R¹ ausgewählt ist aus Aryl, Heteroaryl, Alkyl, Perfluoralkyl, Perfluoraryl, wobei
R² und R³ gleich oder unterschiedlich sind und unabhängig voneinander ausgewählt sind aus Aryl, Heteroaryl, Alkyl, Alkenyl, Alkinyl, Perfluoralkyl, Perfluoraryl, wobei
R⁴, R⁵, R⁶, R⁷, R⁸, R⁹, R¹⁰, R¹¹ gleich oder unterschiedlich sind und unabhängig voneinander ausgewählt sind aus Wasserstoff, Deuterium, Halogen, Silyl, Alkyl, Alkenyl, Alkinyl, Perfluoralkyl, Aryl, Heteroaryl, Alkoxy, wobei
R¹² R¹³, R¹⁴, R¹⁵, R¹⁶, R¹⁷ gleich oder unterschiedlich sind und unabhängig voneinander ausgewählt sind aus Wasserstoff, Deuterium, Halogen, Alkyl, Alkenyl, Alkinyl, Perfluoralkyl, Aryl, Heteroaryl, Alkoxy, COR¹⁸, CO₂R¹⁹, CONR²⁰, Cyano, Nitro, SO₃R²¹, PO₃R²²₂, wobei R¹⁸ R¹⁹, R²⁰, R²¹, R²² gleich oder unterschiedlich sind und unabhängig voneinander ausgewählt sind aus Wasserstoff, Deuterium, Halogen, Silyl, Alkyl, Alkenyl, Alkinyl, Perfluoralkyl, Aryl, Heteroaryl, Alkoxy, und wobei
An⁻ ein Anion ist.

2. Paracyclophan-Carben-Verbindung nach Anspruch 1, **dadurch gekennzeichnet, dass** R¹ ausgewählt ist aus C5 bis C18 Aryl, C5 bis C18 Heteroaryl, C1 bis C18 Alkyl, C1 bis C18 Perfluoralkyl, C5 bis C18 Perfluoraryl, wobei
R² und R³ gleich oder unterschiedlich sind und unabhängig voneinander ausgewählt sind aus C5 bis C18 Aryl, C5 bis C18 Perfluoraryl, C5 bis C18 Heteroaryl, C1 bis C12 Alkyl, C1 bis C12 Alkenyl, C1 bis C12 Alkinyl, C1 bis C12 Perfluoralkyl, wobei
R⁴, R⁵, R⁶, R⁷, R⁸, R⁹, R¹⁰, R¹¹ gleich oder unterschiedlich sind und unabhängig voneinander ausgewählt sind aus Wasserstoff, Deuterium, Halogen, Silyl, C1 bis C12 Alkyl, C1 bis C12 Alkenyl, C1 bis C12 Alkinyl und C1 bis C12 Perfluoralkyl, C5 bis C18 Aryl, C5 bis C18 Perfluoraryl, C5 bis C18 Heteroaryl, C1 bis C12 Alkoxy, wobei
R¹² R¹³, R¹⁴, R¹⁵, R¹⁶, R¹⁷ gleich oder unterschiedlich sind und unabhängig voneinander ausgewählt sind aus Wasserstoff, C1 bis C18 Alkyl, C1 bis C18 Alkenyl, C1 bis C18 Alkinyl, und C1 bis C18 Perfluoralkyl, C5 bis C18 Aryl, C5 bis C18 Perfluoraryl, C5 bis C18 Heteroaryl, C1 bis C18 Alkoxy, COR¹⁸, CO₂R¹⁹, CONR²⁰, Cyano, Nitro, SO₃R²¹, PO₃R²²₂, wobei
R¹⁸ R¹⁹, R²⁰, R²¹, R²² gleich oder unterschiedlich sind und unabhängig voneinander ausgewählt sind aus Wasserstoff, Deuterium, Halogen, Silyl, C1 bis C18 Alkyl, C1 bis C18 Alkenyl, C1 bis C18 Alkinyl, C1 bis C18 Perfluoralkyl, C5 bis C18 Aryl, C5 bis C18 Heteroaryl, C1 bis C18 Alkoxy, und wobei
An⁻ ein Anion ist, das ausgewählt ist aus Halogenid, Triflat, Sulfat, Phosphat, Silikat, Tetrafluoroborat, Hexafluorophosphat, Tetraarylborat.

3. Paracyclophan-Carben-Verbindung nach Anspruch 1 oder 2, **dadurch gekennzeichnet, dass**
R¹ ausgewählt ist aus C9 bis C18 Aryl, C9 bis C18 Heteroaryl, C1 bis C12 Alkyl, C1 bis C12 Perfluoralkyl, C9 bis C18 Perfluoraryl, wobei
R² und R³ gleich oder unterschiedlich sind und unabhängig voneinander ausgewählt sind aus C5 bis C6 Aryl und C5 bis C6 Heteroaryl,
R⁴, R⁵, R⁶, R⁷, R⁸, R⁹, R¹⁰, R¹¹ gleich oder unterschiedlich sind und unabhängig voneinander ausgewählt sind aus Wasserstoff, Deuterium, Halogen und Silyl, C1 bis C4 Alkyl, C1 bis C4 Alkenyl, C1 bis C4 Alkinyl und C1 bis C4 Perfluoralkyl, C5 bis C6 Aryl, C5 bis C6 Heteroaryl, C5 bis C6 Perfluoraryl, C1 bis C4 Alkoxy, wobei
R¹² R¹³, R¹⁴, R¹⁵, R¹⁶, R¹⁷ gleich oder unterschiedlich sind und unabhängig voneinander ausgewählt sind aus Wasserstoff, C1 bis C4 Alkyl, C1 bis C4 Alkenyl, C1 bis C4 Alkinyl, und C1 bis C4 Perfluoralkyl, C5 bis C6 Aryl, C5 bis C6 Perfluoraryl, C5 bis C6 Heteroaryl, C1 bis C4 Alkoxy, COR¹⁸, CO₂R¹⁹, CONR²⁰, Cyano, Nitro, SO₃R²¹, PO₃R²²₂, wobei R¹⁸ R¹⁹, R²⁰, R²¹, R²² gleich oder unterschiedlich sind und unabhängig voneinander ausgewählt sind aus Wasserstoff, Deuterium, Halogen, Silyl, C1 bis C4 Alkyl, C1 bis C4 Alkenyl, C1 bis C4 Alkinyl, C1 bis C4 Perfluoralkyl, C5 bis C12 Aryl, C5 bis C12 Heteroaryl, C1 bis C4 Alkoxy, und wobei
An⁻ ein Anion ist, das ausgewählt ist aus Halogenid, Triflat, Sulfat, Phosphat, Silikat, Tetrafluoroborat, Hexafluorophosphat, Tetraarylborat.

4. Paracyclophan-Carben-Verbindung nach einem der Ansprüche 1 bis 3, **dadurch gekennzeichnet, dass** R¹ Dialkylphenyl ist.

5. Paracyclophan-Carben-Verbindung nach einem der Ansprüche 1 bis 4, **dadurch gekennzeichnet, dass** R² und R³ Phenyl sind.

6. Paracyclophan-Carben-Verbindung nach einem der Ansprüche 1 bis 5, **dadurch gekennzeichnet, dass** R⁴, R⁵, R⁶, R⁷, R⁸, R⁹, R¹⁰ und R¹¹ und/oder dass R¹² R¹³, R¹⁴, R¹⁵, R¹⁶ und R¹⁷ Wasserstoff sind.

7. Paracyclophan-Carben-Verbindung nach einem der Ansprüche 1 bis 6, **dadurch gekennzeichnet, dass** die Paracyclophan-Carben-Verbindung die folgende Struktur (II) aufweist:

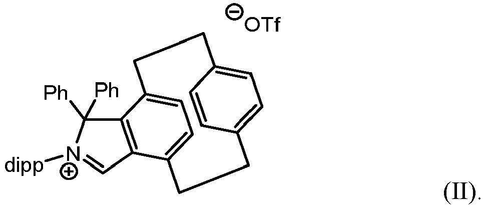

8. Verwendung einer Paracyclophan-Carben-Verbindung nach einem der Ansprüche 1 bis 7 zur Herstellung eines Metallkomplexes für ein Emittermaterial einer Emitterschicht einer organischen Leuchtdiode.

9. Metallkomplex, **dadurch gekennzeichnet, dass** der Metallkomplex der folgenden Struktur (III) entspricht:
L¹ME[L²],ₘ[L^{3]}ₘ (III),
wobei L¹ ein Paracyclophan-Carben-Ligand nach einem der Ansprüche 1 bis 8 ist, wobei L² und L³ gleich oder unterschiedlich sind und unabhängig voneinander ausgewählt sind aus NR²³₂, NR²⁴₃, PR²⁵₂, PR²⁶₃, OR²⁷₂, OR²⁸, SR²⁹, SR³⁰₂, SR³¹, SeR³²₂, SeR³⁴, TeR³⁵₂, TeR³⁶, Silyl, Aryl, Heteroaryl, Alkenyl, Alkinyl, Isonitril, Cyclopentadienyl, Halogen, wobei m eine Zahl ist ausgewählt aus 0, 1 oder 2 mit der Maßgabe, dass wenigstens ein m ungleich null ist, wobei
R²³, R²⁴, R²⁵, R²⁶, R²⁷, R²⁸, R²⁹, R³⁰, R³¹, R³², R³³, R³⁴, R³⁵, R³⁶ gleich oder unterschiedlich sind und unabhängig voneinander ausgewählt sind aus Aryl, Heteroaryl, Alkenyl, Alkinyl, Alkyl, Perfluoralkyl, Perfluoraryl; und wobei
Me ein Metall ist, das ausgewählt ist aus Kupfer, Silber und Gold.

10. Metallkomplex nach Anspruch 9, **dadurch gekennzeichnet, dass** L¹ ein Paracyclophan-Carben-Ligand nach einem der Ansprüche 1 bis 7 ist, wobei Me ein Metall ist, das ausgewählt ist aus Kupfer, Silber und Gold, und wobei L² ein Ligand ist, der ausgewählt ist aus einem Heteroaryl und einem Halogen
oder
dass Me Kupfer ist, wobei L² ein Ligand ist, der ausgewählt ist aus einem Carbazol-Liganden und einem Halogen.

11. Metallkomplex nach einem der Ansprüche 9 bis 10, **dadurch gekennzeichnet, dass** der Metallkomplex ausgewählt ist aus den Strukturen (IV), (V), (VI), (VII), (VIII) und (IX) wie nachfolgend gezeigt:

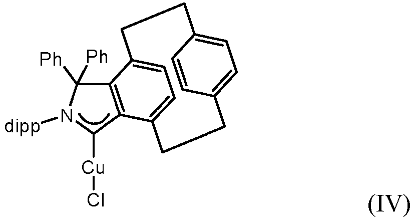

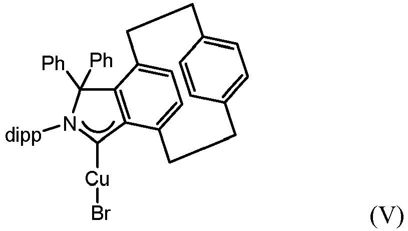

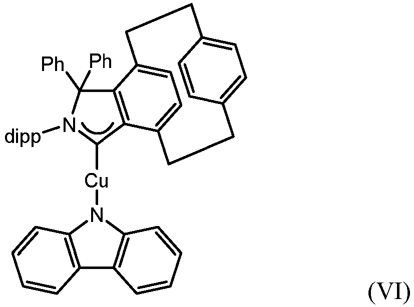

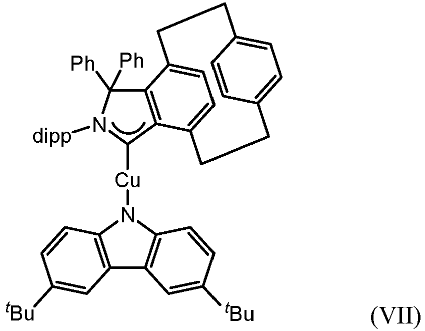

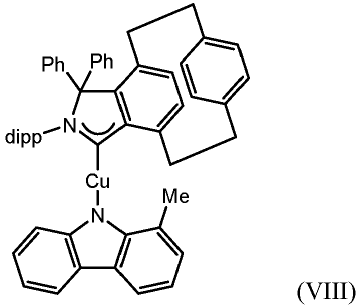

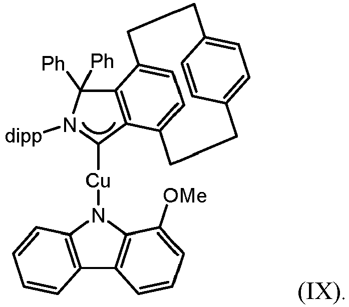

12. Verwendung eines Metallkomplexes als Emittermaterial in einer Emitterschicht einer organischen Leuchtdiode, **dadurch gekennzeichnet**, der Metallkomplex ausgebildet ist nach einem der Ansprüche 9 bis 11.

13. Emitterschicht für eine organische Leuchtdiode, aufweisend einen Metallkomplex zum Emittieren von Licht, **dadurch gekennzeichnet, dass** der Metallkomplex ausgebildet ist nach einem der Ansprüche 9 bis 11.

14. Organische Leuchtdiode, aufweisend eine Kathode, eine Emitterschicht, eine Lochleitungsschicht und eine Anode, **dadurch gekennzeichnet, dass** die Emitterschicht ausgebildet ist nach Anspruch 13 und optional weiterhin einen Filter zum Reduzieren von Reflektionen externer Lichtquellen aufweist, wobei der Filter zumindest zum Teil undurchlässig ist für linear polarisiertes Licht.

15. Verfahren zum Erzeugen von Licht durch eine organische Leuchtdiode, aufweisend die Verfahrensschritte:
a) Bereitstellen einer organischen Leuchtdiode nach Anspruch 14, und
b) Anlegen einer Spannung zwischen Anode und Kathode zur Injektion von Ladungsträgern und zur Erzeugung von Licht.

## Claims

1. A paracyclophane-carbene compound, **characterized in that** the paracyclophane-carbene compound corresponds to the following structure (I): 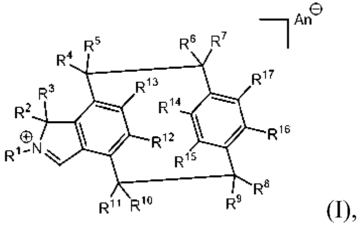 wherein
R¹ is selected from aryl, heteroaryl, alkyl, perfluoroalkyl, perfluoroaryl, wherein
R² and R³ are the same or different and are independently selected from aryl, heteroaryl, alkyl, alkenyl, alkynyl, perfluoroalkyl, perfluoroaryl, wherein
R⁴, R⁵, R⁶, R⁷, R⁸, R⁹, R¹⁰, R¹¹ are the same or different and are independently selected from hydrogen, deuterium, halogen, silyl, alkyl, alkenyl, alkynyl, perfluoroalkyl, aryl, heteroaryl, alkoxy, wherein
R¹², R¹³, R¹⁴, R¹⁵, R¹⁶, R¹⁷ are the same or different and are independently selected from hydrogen, deuterium, halogen, alkyl, alkenyl, alkynyl, perfluoroalkyl, aryl, heteroaryl, alkoxy, COR¹⁸, CO₂R¹⁹, CONR²⁰, cyano, nitro, SO₃R²¹, PO₃R²²₂, wherein
R¹⁸, R¹⁹, R²⁰, R²¹, R²² are the same or different and are independently selected from hydrogen, deuterium, halogen, silyl, alkyl, alkenyl, alkynyl, perfluoroalkyl, aryl, heteroaryl, alkoxy, and wherein
An⁻ is an anion.

2. Paracyclophane-carbene compound according to claim 1, **characterised in that** R¹ is selected from C5 to C18 aryl, C5 to C18 heteroaryl, C1 to C18 alkyl, C1 to C18 perfluoroalkyl, C5 to C18 perfluoroaryl, wherein
R² and R³ are the same or different and are independently selected from C5 to C18 aryl, C5 to C18 perfluoroaryl, C5 to C18 heteroaryl, C1 to C12 alkyl, C1 to C12 alkenyl, C1 to C12 alkynyl, C1 to C12 perfluoroalkyl, wherein
R⁴, R⁵, R⁶, R⁷, R⁸, R⁹, R¹⁰, R¹¹ are the same or different and are independently selected from hydrogen, deuterium, halogen, silyl, C1 to C12 alkyl, C1 to C12 alkenyl, C1 to C12 alkynyl and C1 to C12 perfluoroalkyl, C5 to C18 aryl, C5 to C18 perfluoroaryl, C5 to C18 heteroaryl, C1 to C12 alkoxy, wherein
R¹² R¹³, R¹⁴, R¹⁵, R¹⁶, R¹⁷ are the same or different and are independently selected from hydrogen, C1 to C18 alkyl, C1 to C18 alkenyl, C1 to C18 alkynyl, and C1 to C18 perfluoroalkyl, C5 to C18 aryl, C5 to C18 perfluoroaryl, C5 to C18 heteroaryl, C1 to C18 alkoxy, COR¹⁸, CO₂R¹⁹, CONR²⁰, cyano, nitro, SO₃R²¹, PO₃R²²₂, wherein
R¹⁸ R¹⁹, R²⁰, R²¹, R²² are the same or different and are independently selected from hydrogen, deuterium, halogen, silyl, C1 to C18 alkyl, C1 to C18 alkenyl, C1 to C18 alkynyl, C1 to C18 perfluoroalkyl, C5 to C18 aryl, C5 to C18 heteroaryl, C1 to C18 alkoxy, and wherein
An⁻ is an anion selected from halide, triflate, sulphate, phosphate, silicate, tetrafluoroborate, hexafluorophosphate, tetraarylborate.

3. Paracyclophane-carbene compound according to claim 1 or 2, **characterized in that** R¹ is selected from C9 to C18 aryl, C9 to C18 heteroaryl, C1 to C12 alkyl, C1 to C12 perfluoroalkyl, C9 to C18 perfluoroaryl, wherein
R² und R³ are the same or different and are independently selected from C5 to C6 aryl and C5 to C6 heteroaryl,
R⁴, R⁵, R⁶, R⁷, R⁸, R⁹, R¹⁰, R¹¹ are the same or different and are independently selected from hydrogen, deuterium, halogen and silyl, C1 to C4 alkyl, C1 to C4 alkenyl, C1 to C4 alkinyl and C1 to C4 perfluoroalkyl, C5 to C6 aryl, C5 to C6 heteroaryl, C5 to C6 perfluoroaryl, C1 to C4 alkoxy, wherein
R¹² R^{13,} R¹⁴, R¹⁵, R¹⁶, R¹⁷ are the same or different and are independently selected from hydrogen, C1 to C4 alkyl, C1 to C4 alkenyl, C1 to C4 alkynyl, and C1 to C4 perfluoroalkyl, C5 to C6 aryl, C5 to C6 perfluoroaryl, C5 to C6 heteroaryl, C1 to C4 alkoxy, COR¹⁸, CO₂R¹⁹, CONR²⁰, cyano, nitro, SO₃R²¹, PO₃R²²₂, wherein
R¹⁸ R¹⁹, R²⁰, R²¹, R²² are the same or different and are independently selected from hydrogen, deuterium, halogen, silyl, C1 to C4 alkyl, C1 to C4 alkenyl, C1 to C4 alkynyl, C1 to C4 perfluoroalkyl, C5 to C12 aryl, C5 to C12 heteroaryl, C to C4 alkoxy, and wherein
An⁻ is an anion selected from halide, triflate, sulphate, phosphate, silicate, tetrafluoroborate, hexafluorophosphate, tetraarylborate.

4. Paracyclophane-carbene compound according to any one of claims 1 to 3, **characterized in that** R¹ is dialkylphenyl.

5. Paracyclophane-carbene compound according to any one of claims 1 to 4, **characterized in that** R² and R³ are phenyl.

6. Paracyclophane-carbene compound according to any one of claims 1 to 5, **characterized in that** R⁴, R⁵, R ⁶ , R⁷, R⁸, R⁹, R¹⁰ and R¹¹ and/or that R¹² R₁₃, R¹⁴, R¹⁵, R¹⁶ and R¹⁷ are hydrogen.

7. Paracyclophane-carbene compound according to any one of claims 1 to 6, **characterized in that** the paracyclophane-carbene compound has the following structure (II):

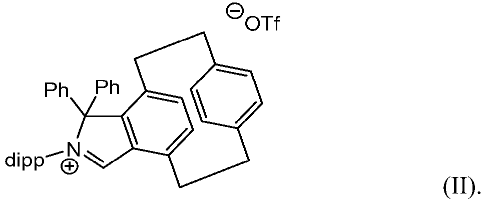

8. Use of a paracyclophane-carbene compound according to any one of claims 1- 7 for preparing a metal complex for an emitter material of an emitter layer of an organic light-emitting diode.

9. Metal complex, **characterized in that** the metal complex corresponds to the following structure (III):
L¹Me[L²]ₘ[L³]ₘ (III),
wherein L¹ is a paracyclophane-carbene ligand according to any one of claim 1 to 8, wherein L² and L³ are the same or different and are independently selected from NR²³₂, NR²⁴₃, PR²⁵₂, PR²⁶₃, OR²⁷₂, OR²⁸, SR²⁹, SR³⁰₂, SR³¹, SeR³²₂, SeR³⁴, TeR³⁵₂, TeR³⁶, silyl, aryl, heteroaryl, alkenyl, alkynyl, isonitrile, cyclopentadienyl, halogen, wherein
m is a number selected from 0, 1 or 2, with the proviso that at least one m is not equal to zero, wherein
R²³, R²⁴, R²⁵, R²⁶, R²⁷, R²⁸, R²⁹, R³⁰, R³¹, R³², R³³, R³⁴, R³⁵, R³⁶ are the same or different and are independently selected from aryl, heteroaryl, alkenyl, alkynyl, alkyl, perfluoroalkyl, perfluoroaryl; and wherein
Me is a metal selected from copper, silver and gold.

10. Metal complex according to claim 9, **characterized in that**
L¹ is a paracyclophane-carbene ligand according to any one of claims 1 to 7, wherein
Me is a metal selected from copper, silver and gold, and wherein
L² is a ligand selected from a heteroaryl and a halogen
or
that Me is copper, wherein L² is a ligand selected from a carbazole-ligand and a halogen.

11. Metal complex according to any one of claim 9 to 10, **characterized in that** the metal complex is selected from the structures (IV), (V), (VI), (VII), (VIII) and (IX) as shown below:

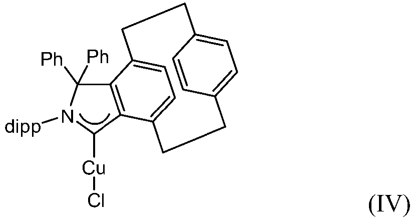

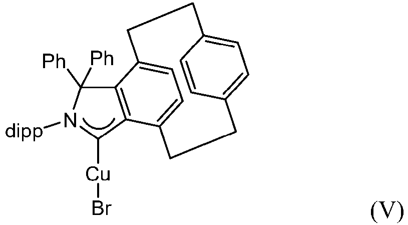

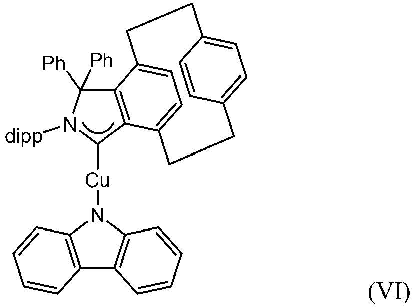

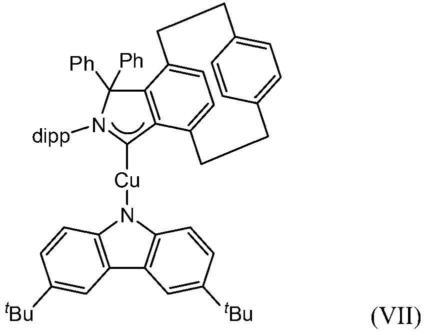

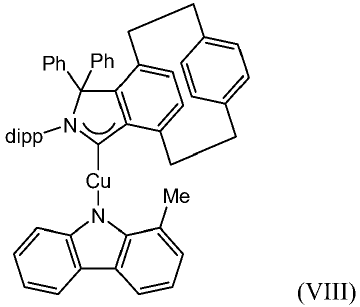

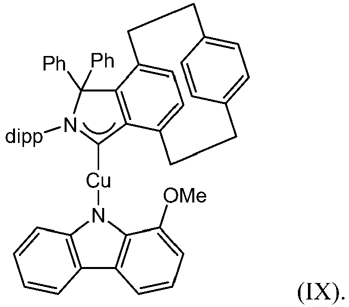

12. Use of a metal complex as emitter material in an emitter layer of an organic light-emitting diode, **characterized in that** the metal complex is formed according to any one of claims 9 to 11.

13. Emitter layer for an organic light-emitting diode, having a metal complex for emitting light, **characterized in that** the metal complex is formed according to any one of claims 9 to 11.

14. Organic light-emitting diode comprising a cathode, an emitter layer, a hole transport layer and an anode, **characterized in that** the emitter layer is formed according to claim 13 and, optionally, the presence of a filter for the reduction of reflexions from external light sources, wherein the filter is at least partially not permeable for polarized light.

15. A method for generating light by means of an organic light-emitting diode, comprising the steps of:
a) providing an organic light-emitting diode according to claim 14, and
b) applying a voltage between the anode and the cathode to inject charge carriers and generate light.

## Revendications

1. Composé paracyclophane-carbène, **caractérisé en ce que** le composé paracyclophane-carbène correspond à la structure (I) suivante: 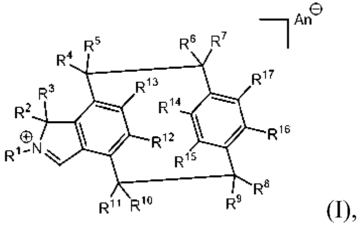 dans laquelle
R¹ est sélectionné parmi les groupes aryle, hétéroaryle, alkyle, perfluoroalkyle et perfluoroaryle, dans lequelle
R² et R³ sont identiques ou différents et sont sélectionnés indépendamment les uns des autres parmi les groupes aryle, hétéroaryle, alkyle, alcényle, alcynyle, perfluoroalkyle et perfluoroaryle, dans laquelle
R⁴, R⁵, R⁶, R⁷, R⁸, R⁹, R¹⁰, R¹¹ sont identiques ou différents et sont choisis indépendamment les uns des autres parmi l'hydrogène, le deutérium, un halogène, les groupes silyle, alkyle, alcényle, alcynyle, perfluoroalkyle, aryle, hétéroaryle, alcoxyle, dans laquelle
R¹² R¹³, R¹⁴, R¹⁵, R ¹⁶, R¹⁷ sont identiques ou différents et sont choisis indépendamment les uns des autres parmi l'hydrogène, le deutérium, un halogène, les groupes alkyle, alcényle, alcynyle, perfluoroalkyle, aryle, hétéroaryle, alcoxyle, COR¹⁸, CO₂R¹⁹, CONR²⁰, cyano, nitro, SO₃R²¹, PO₃R²²₂, dans laquelle
R¹⁸ R¹⁹, R²⁰, R²¹, R²² sont identiques ou différents et sont choisis indépendamment les uns des autres parmi l'hydrogène, le deutérium, un halogène, les groupes silyle, alkyle, alcényle, alcynyle, perfluoroalkyle, aryle, hétéroaryle, alcoxyle, et dans laquelle An⁻ c'est un anion.

2. Composé paracyclophane-carbène selon la revendication 1, **caractérisé en ce que**
R¹ est sélectionné à partir les groupes aryle en C5 à C18, hétéroaryle en C5 à C18, alkyle en C1 à C18, perfluoroalkyle en C1 à C18, perfluoroalkyle en C5 à C18, dans lequel
R² et R³ sont identiques ou différents et sont sélectionnés indépendamment les uns des autres parmi les groupes aryle en C5 à C18, perfluoroaryle en C5 à C18, hétéroaryle en C5 à C18, alkyle en C1 à C12, alcényle en C1 à C12, alcynyle en C1 à C12, perfluoroalkyle en C1 à C12, dans lequel
R⁴, R⁵, R⁶, R⁷, R⁸, R⁹, R¹⁰, R¹¹ sont identiques ou différents et sont sélectionnés indépendamment les uns des autres parmi l'hydrogène, le deutérium, un halogène, les groupes silyle, alkyle en C1 à C12, alcényle en C1 à C12, alcynyle en C1 à C12, perfluoroalkyle en C1 à C12, aryle en C5 à C18, perfluoroaryle en C5 à C18, hétéroaryle en C5 à C18, alcoxyle en C1 à C12, dans lequel
R¹² R¹³, R¹⁴, R¹⁵, R¹⁶, R¹⁷ sont identiques ou différents et sont choisis indépendamment les uns des autres parmi l'hydrogène, les groupes alkyle en C1 à C18, alcényle en C1 à C18, alcynyle en C1 à C18, et perfluoroalkyle en C1 à C18, aryle en C5 à C18, perfluoroaryle en C5 à C18, hétéroaryle en C5 à C18, alcoxyle en C1 à C18, COR¹⁸, CO₂R¹⁹, CONR²⁰, cyano, nitro, SO₃R²¹, PO₃ R ²²₂, dans lequel
R¹⁸ R¹⁹, R²⁰, R²¹, R²² sont identiques ou différents et sont sélectionnés indépendamment les uns des autres parmi l'hydrogène, le deutérium, un halogène, les groupes silyle, alkyle en C1 à C18, alcényle en C1 à C18, alcynyle en C1 à C18, perfluoroalkyle en C1 à C18, aryle en C5 à C18, hétéroaryle en C5 à C18, alcoxyle en C1 à C18, et dans lequel
An⁻ est un anion sélectionné parmi l'halogénure, le triflate, le sulfate, le phosphate, le silicate, le tétrafluoroborate, l'hexafluorophosphate, le tétraarylborate.

3. Composé paracyclophane-carbène selon la revendication 1 ou 2, **caractérisé en ce que** R¹ est sélectionné parmi les groupes aryle en C9 à C18, hétéroaryle en C9 à C18, alkyle en C1 à C12, perfluoroalkyle en C1 à C12, perfluoroaryle en C9 à C18, dans lequel
R² et R³ sont identiques ou différents et sont sélectionnés indépendamment l'un de l'autre parmi les groupes aryle en C5 à C6 et hétéroaryle en C5 à C6,
R⁴, R⁵, R⁶, R⁷, R⁸, R⁹, R¹⁰, R¹¹ sont identiques ou différents et sont sélectionnés indépendamment les uns des autres parmi l'hydrogène, le deutérium, un halogène et les groupes silyle, alkyle en C1 à C4, alcényle en C1 à C4, alcynyle en C1 à C4 et perfluoroalkyle en C1 à C4, aryle en C5 à C6, hétéroaryle en C5 à C6, perfluoroaryle en C5 à C6, alcoxyle en C1 à C4, dans lequel
R¹² R¹³, R¹⁴, R¹⁵, R¹⁶, R¹⁷ sont identiques ou différents et sélectionnés indépendamment les uns des autres parmi l'hydrogène, les groupes alkyle en C1 à C4, alcényle en C1 à C4, alcynyle en C1 à C4, et perfluoroalkyle en C1 à C4, aryle en C5 à C6, perfluoroaryle en C5 à C6, hétéroaryle en C5 à C6, alcoxyle en C1 à C4, COR¹⁸, CO₂R¹⁹, CONR²⁰, cyano, nitro, SO₃R²¹, PO₃ R²²₂, dans lequel
R¹⁸ R¹⁹, R²⁰, R²¹, R²² sont identiques ou différents et sont sélectionnés indépendamment les uns des autres parmi l'hydrogène, le deutérium, un halogène, les groupes silyle, alkyle en C1 à C4, alcényle en C1 à C4, alcynyle en C1 à C4, perfluoroalkyle en C1 à C4, aryle en C5 à C12, hétéroaryle en C5 à C12, alcoxyle en C1 à C4, et dans lequel
An⁻ est un anion sélectionné parmi l'halogénure, le triflate, le sulfate, le phosphate, le silicate, le tétrafluoroborate, l'hexafluorophosphate, le tétraarylborate.

4. Composé paracyclophane-carbène selon l'une des revendications 1 à 3, **caractérisé en ce que** R¹ est un groupe dialkylphényle.

5. Composé paracyclophane-carbène selon l'une des revendications 1 à 4, **caractérisé en ce que** R² et R³ sont un groupe phényle.

6. Composé paracyclophane-carbène selon l'une des revendications 1 à 5, **caractérisé en ce que** R⁴, R⁵, R⁶, R⁷, R⁸, R⁹, R¹⁰ et R¹¹ et/ou R¹² R¹³, R ¹⁴, R¹⁵, R ¹⁶ et R¹⁷ sont de l'hydrogène.

7. Composé paracyclophane-carbène selon l'une des revendications 1 à 6, **caractérisé en ce que** le composé paracyclophane-carbène présente la structure (II) suivante:

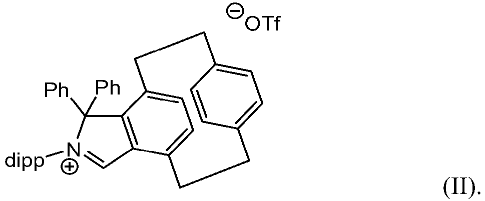

8. Utilisation d'un composé paracyclophane-carbène selon l'une des revendications 1 à 7 pour la fabrication d'un complexe métallique destiné à un matériau émetteur d'une couche émettrice d'une diode électroluminescente organique.

9. Complexe métallique **caractérisé en ce que** le complexe métallique correspond à la structure (III) suivante:
L¹Me[L²]ₘ[L³]ₘ (III),
dans lequel L¹ est un ligand de carbène paracyclophane selon l'une des revendications 1 à 8, dans lequel
L² et L³ sont identiques ou différents et sont sélectionnés indépendamment l'un de l'autre parmi NR²³₂, NR²⁴₃, PR²⁵₂, PR²⁶₃, OR²⁷₂, OR²⁸, SR²⁹, SR ³⁰₂, SR³¹, SeR³²₂, SeR ³⁴, TeR³⁵₂, TeR³⁶, les groupes silyle, aryle, hétéroaryle, alcényle, alcynyle, un isonitrile, un groupe cyclopentadiényle, un halogène, dans lequel
m est un nombre sélectionné parmi 0, 1 ou 2, à condition qu'au moins un m est différent de zéro, dans lequel
R²³, R²⁴, R²⁵, R²⁶, R²⁷, R²⁸, R²⁹, R³⁰, R³¹, R³², R³³, R³⁴, R³⁵, R³⁶ sont identiques ou différents et sont sélectionnés indépendamment les uns des autres parmi les groupes aryle, hétéroaryle, alcényle, alcynyle, alkyle, perfluoroalkyle, perfluoroaryle; et dans lequel Me est un métal qui est sélectionné parmi le cuivre, l'argent et l'or.

10. Complexe métallique selon la revendication 9, **caractérisé en ce que** L¹ est un ligand de paracyclophane-carbène selon l'une des revendications 1 à 7, dans lequel
Me est un métal qui est sélectionné parmi le cuivre, l'argent et l'or, et dans lequel
L² est un ligand sélectionné parmi un groupe hétéroaryle et un halogène
ou
Me est du cuivre, dans lequel L² est un ligand sélectionné parmi un ligand carbazole et un halogène.

11. Complexe métallique selon l'une des revendications 9 à 10, **caractérisé en ce que** le complexe métallique est sélectionné parmi les structures (IV), (V), (VI), (VII), (VIII) et (IX) comme indiqué ci-dessous:

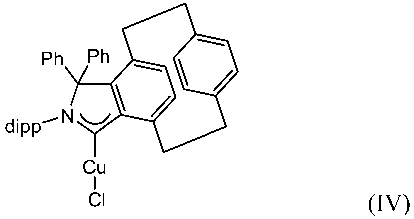

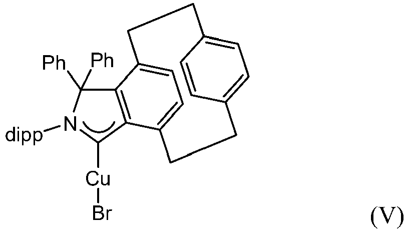

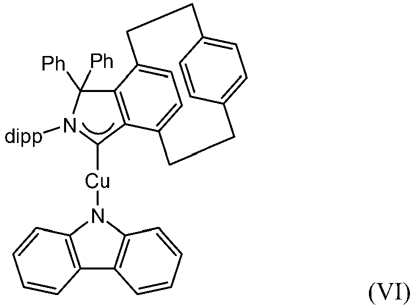

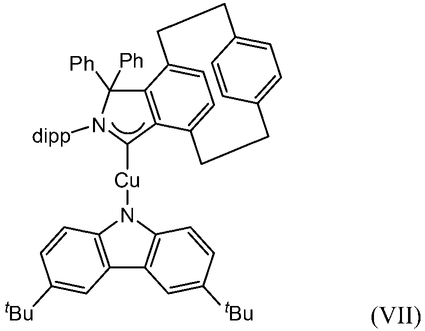

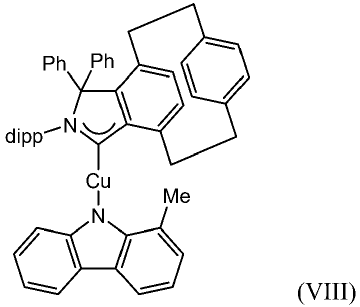

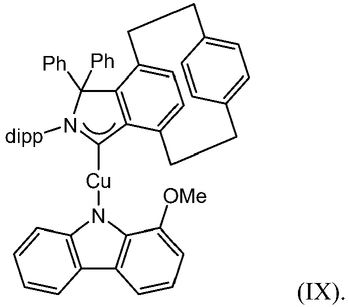

12. Utilisation d'un complexe métallique comme matériau émetteur dans une couche émettrice d'une diode électroluminescente organique, **caractérisée par** ce que le complexe métallique est conçu selon l'une des revendications 9 à 11.

13. Couche émettrice pour diode électroluminescente organique comprenant un complexe métallique destiné à émettre de la lumière, **caractérisée en ce que** le complexe métallique est conçu selon l'une des revendications 9 à 11.

14. Diode électroluminescente organique comprenant une cathode, une couche émettrice, une couche conductrice de trous et une anode, **caractérisée en ce que** la couche émettrice est conçue selon la revendication 13 et comprend en option un filtre permettant de réduire les reflets de sources lumineuses externes, dans laquelle le filtre étant au moins partiellement imperméable à la lumière linéairement polarisée.

15. Procédé de production de lumière à l'aide une diode électroluminescente organique, comprenant les étapes du procédé:
A) fourniture d'une diode électroluminescente organique selon la revendication 14; et
B) application d'une tension entre l'anode et la cathode pour injecter des porteurs de charge et produire de la lumière.
